# EUROPÄISCHE PATENTSCHRIFT

(11) **EP 3 574 852 B1**
(45) Veröffentlichungstag und Bekanntmachung des Hinweises auf die Patenterteilung: **09.09.2020**
(21) Anmeldenummer: 19167014.0
(22) Anmeldetag: 03.04.2019
(51) Int. Cl.: A61B 17/14, A61B 17/16, A61B 17/32

(54) **VAKUUMMOTOR, CHIRURGISCHES ANTRIEBSSYSTEM UND VERFAHREN ZUM BETREIBEN EINES VAKUUMMOTORS**
VACUUM MOTOR, SURGICAL DRIVE SYSTEM AND METHOD FOR OPERATING A VACUUM MOTOR
MOTEUR À VIDE, SYSTÈME D'ENTRAÎNEMENT CHIRURGICAL ET PROCÉDÉ DE FONCTIONNEMENT D'UN MOTEUR À VIDE

(30) Priorität: 30.05.2018 DE 102018208567
(43) Veröffentlichungstag der Anmeldung: 04.12.2019
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- US-B2- 9 861 770

## Beschreibung

Die Erfindung betrifft einen Vakuummotor, ein chirurgisches Antriebssystem mit einem solchen Vakuummotor, ein medizinisches Lavage-System zum Debridement von Weichgewebe und/oder Knochengewebe aufweisend einen solchen Vakuummotor und eine medizinische Vorrichtung zum Bürsten, Raspeln oder Sägen von Weichgewebe und/oder Knochengewebe mit einem solchen Vakuummotor sowie ein Verfahren zum Betreiben eines Vakuummotors. Der Vakuummotor ist zum Antrieb von medizinischen Vorrichtungen für das Lavagieren und das Debridement von Weich- und Knochengewebe geeignet. Die mit dem Vakuummotor angetriebenen medizinischen Vorrichtungen sind vorzugsweise aus hygienischen Gründen zum einmaligen Gebrauch bestimmt. Weiterhin wird ein Verfahren zum Erzeugen von oszillierenden, linearen Bewegungen vorgeschlagen.

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Das Debridieren kann durch Wundspülung mit sogenannten Lavage-Systemen sowie durch Ausschneiden, Raspeln, Sägen und auch Bürsten erfolgen. Die zum Debridieren verwendeten Vorrichtungen sind nach dem Debridement mit Geweberesten und mit mikrobiellen Keimen kontaminiert. Für eine neue Verwendung müssen diese Instrumente sorgfältig gereinigt und anschließend sterilisiert werden. Dabei muss sich das medizinische Personal vor einer Kontamination beziehungsweise einer Infektion durch Übertragung von mikrobiellen Keimen während der Reinigungsarbeiten schützen. Daher ist es wünschenswert, wenn eine kostengünstige Vorrichtung mit Motorantrieb zum Raspeln, Sägen und Bürsten für septische Revisionen bereitgestellt werden könnte, die nach einmaliger Verwendung ohne aufwendige und nicht gefahrlose Reinigungsschritte mit dem üblichen OP-Abfall zusammen entsorgt werden könnte. Es wäre dann besonders aus Gründen des Ressourcen- und Umweltschutzes aber auch aus Kostengründen sinnvoll, wenn für den Antrieb keine Batterien, Akkumulatoren und Elektromotoren notwendig wären.

Mit Lavage-Systemen werden mit den Spülflüssigkeiten Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506; S. J. Breusch et al.: Lavage technique in THA: Jet-lavage Produces Better Cement Penetration Than Syringe-Lavage in the Proximal Femur. J. Arthroplasty. 200; 15(7): 921-927; R. J. Byrick et al.: High-volume, high pressure pulsatile lavage during cemented arthroplasty. J. Bone Joint Surg. 1989; 81-A: 1331-1336; J. Christie et al.: Medullary lavage reduces embolic phenomena and cardiopulmonary changes during cemented hemiarthorplasty. J. Bone Joint Surg. 1995; 77-B: 456-459). Gepulste Lavage-Systeme sind seit langem bekannt, beispielsweise aus der US 4 583 531 A, der US 4 278 078 A und der US 5 542 918 A.

Bei der Verwendung von Druckluft-getriebenen Lavage-Systemen muss jedoch ein Doppelschlauchsystem verwendet werden, bei dem in einem Schlauch die unsterile Druckluft zugeführt wird und einen zweiten Schlauch mit dem die nach dem Antrieb des Druckluftmotors zumindest teilentspannte unsterile Luft abgeführt wird. Bei den mit Druckluft oder einem anderen komprimierten Gas getriebenen Systemen wird üblicherweise ein Druckgasmotor zum Antrieb eingesetzt. Ein solcher Druckgasmotor ist aus der WO 2012/038003 A1 bekannt. Der dort beschriebene Druckgasmotor hat einen zweiteiligen Kolben mit einem Zwischenraum und einen Durchgang durch einen der Kolbenteile. Dadurch ist der Motor besonders einfach und kostengünstig im Aufbau. Ein Druckgasmotor für eine Sprühdose ist aus der DE 37 24 110 A1 bekannt. Eine mit Druckgas angetriebene Hubkolbenpumpe ist aus der US 4 993 924 A bekannt. Ein Druckgasmotor für ein Lavage-System ist aus der EP 2 873 856 A1 bekannt. Bei diesem Druckgasmotor wird ein Steuerkolben von einem Arbeitskolben über ein Mitnehmerelement bewegt und dadurch werden im Betrieb des Druckgasmotors eine Gaseinlassöffnung und eine Gasauslassöffnung periodisch geöffnet und geschlossen.

Bei einer Vielzahl von Operationen ist ein Absaugen von Wundsekret und Blut notwendig. Für das Absaugen dieser Flüssigkeiten werden Absaugvorrichtungen eingesetzt, die mit Unterdruck betrieben werden. Für den Betrieb dieser Vorrichtungen gibt es dazu in den meisten Operationssälen (OPs) stationäre Vakuumanlagen, die üblicherweise einen Unterdruck von 0,8 bar bis 0,9 bar bereitstellen. Daneben werden auch mobile Vakuum- beziehungsweise Unterdruck-erzeugende Absauganalgen in breitem Umfang eingesetzt.

Es besteht immer der Wunsch nach einem kostengünstiger aufzubauenden Motor. Zudem besteht auch ein Bedürfnis danach, einen Motor bereitzustellen, der mit einer höheren Frequenz und/oder einer größeren Kraft betrieben werden kann.

Die US 5 554 011 A offenbart eine Pumpe mit einem Vakuummotor, bei dem ein Ventilelement durch die Bewegung einer Membran gesteuert wird. An der Membran wird die Arbeit durch den wechselnden Gasdruck geleistet und dadurch die Pumpe angetrieben. Der Aufbau dieses Vakuummotors ist relativ aufwendig und es kann aufgrund der Membran und der notwendigen Verspannung der Membran bei gleichartig aufgebauten Vakuummotoren zu unterschiedlichem Schwingungsverhalten kommen. Zudem besteht bei dem Ventilelement die Gefahr eines Totpunkts, aus dem Heraus der Vakuummotor nicht mehr selber weiterlaufen kann.

Aus der US 5 542 918 A ist eine Flüssigkeitspumpe für ein Lavage-System bekannt, das von einem Unterdruck angetrieben wird und bei dem eine Membran von einem hohlförmigen, linear beweglichen und federnd gelagerten Kolben angetrieben wird, in dem ein ebenfalls federnd gelagerter Ventilkörper zum Öffnen und Schließen eines Gaseinlasses zum Zuführen von Luft angeordnet ist. Das System hat den Nachteil, dass das Ventilelement im geöffneten Zustand nur einen geringen freien Strömungsquerschnitt bereitstellen kann. Dadurch ist die mit der Pumpe erzeugbare Kraft begrenzt und es besteht die Gefahr eines Totpunkts, aus dem heraus die Pumpe nicht von alleine wieder startet. Zudem kann durch Durchführungen im Gehäuse, die für Stangen zur Verbindung des Kolbens mit der Membran vorgesehen sind, wobei die Stangen durch den an die Unterdruckquelle angeschlossenen Raum laufen, von außen Luft durch die Durchführungen eindringen und dadurch die Leistung der Pumpe weiter reduziert werden, sofern die Stangen nicht aufwendig abgedichtet gelagert werden, was bei einer hohen Arbeitsfrequenz der Pumpe zunehmend schwierig und daher aufwendig ist. Auch kann es durch die fehlende Führung des Ventilkörpers zu seitlichen Bewegungen des Ventilkörpers und dadurch zu unregelmäßigen Schwingungen des Kolbens kommen.

Die US 2005/0084395 A1 offenbart ein vakuumgetriebenes Lavage-System, das über zwei Zylinder arbeitet. In den Zylindern werden zwei miteinander gekoppelte Kolben von dem Vakuum angetrieben.

In den Patenten EP 2 910 270 B1 und US 9 861 770 B2 wird ein Vakuummotor zum Antrieb einer Pumpe für ein Lavage-System beschrieben. Der Vakuummotor besteht aus einem axial in einem Hohlraum beweglichen Arbeitskolben und einem dahinter angeordneten Rückstellelement. Der Arbeitskolben hat ein Mitnehmerelement, das im Betrieb einen Steuerkolben bewegt. Der Steuerkolben ist ein Hohlzylinder und kann über seine geschlossene Mantelfläche eine Gasauslassöffnung und eine Gaseinlassöffnung verdecken. An die Gasauslassöffnung ist eine Unterdruckquelle wie beispielsweise eine Vakuumpumpe angeschlossen. Das bedeutet, dass beim Betrieb des Motors periodisch die Gasauslassöffnung und die Gaseinlassöffnung verdeckt werden. Bei geöffneter Gasauslassöffnung wird die Luft aus dem Vakuummotor gesaugt, wobei der Arbeitskolben gegen das Rückstellelement bewegt wird. Die Zuluftöffnung ist gleichzeitig durch den Steuerkolben verschlossen. Das Rückstellelement wird gespannt. Dann erteilt das Mitnehmerelement dem Steuerkolben einen Impuls. Der Steuerkolben bewegt sich vom Arbeitskolben weg und öffnet die Gaseinlassöffnung und verschließt gleichzeitig die Gasauslassöffnung. Das Vakuum bricht zusammen und das Rückstellelement bewegt den Arbeitskolben in seine Ausgangslage. Dabei erteilt der Mitnehmer dem Steuerkolben einen Impuls. Der Steuerkolben verschiebt sich in Richtung Arbeitskolben. Die Gaseinlassöffnung wird durch die Mantelfläche des Steuerkolbens verschlossen und die Gasauslassöffnung wird geöffnet. Dann beginnt der Zyklus erneut. Dieser Vakuummotor ist für hohe Pulszahlen von ca. 2000 Pulsen pro Minute zum Betrieb einer Pumpe für ein Lavage-System ausgelegt. Die Hublänge ist relativ kurz und beträgt zwischen 2 und 5 mm. Für das Bürsten, Raspeln oder Sägen werden größere Hublängen und geringe Pulszahlen benötigt. Für die Fertigung des in den Patenten EP 2 910 270 B1 und US 9 861 770 B2 beschriebenen Vakuummotors sind durch Kunststoffpräzisionsspritzgießen gefertigte Bauteile notwendig. Die Montage dieser Teile bedarf einer hohen Sorgfalt.

Die Aufgabe der vorliegenden Erfindung besteht also darin, die Nachteile des Stands der Technik zu überwinden. Insbesondere besteht die Aufgabe der Erfindung in der Entwicklung eines Vakuummotors, der kostengünstig und einfach zu fertigen ist, der zuverlässig arbeitet, vielseitig einsetzbar ist und mit Hilfe einer Unterdruckquelle, wie sie in Krankenhäusern zur Verfügung steht, eine ausreichende Leistung erzielt, um Gewebe zu debridieren und hierfür Werkzeuge für die Chirurgie anzutreiben. Daher ist es auch Aufgabe der vorliegenden Erfindung, derartige chirurgische Werkzeuge und Lavage-Systeme mit einem solchen Vakuummotor bereitzustellen. Ebenso ist es Aufgabe der vorliegenden Erfindung ein Verfahren zum Betreiben eines Vakuummotors bereitzustellen, wobei das Verfahren die oben zum Vakuummotor genannten Vorteile bietet.

Aufgabe der Erfindung ist es auch, einen extrem vereinfachten, kostengünstigen Vakuummotor zu entwickeln, der mit Vakuum beziehungsweise Unterdruck angetrieben werden kann und der eine oszillierende, lineare Bewegung erzeugt. Der Vakuummotor soll einfacher aufgebaut und kostengünstiger zu fertigen sein, als der in den Patenten EP 2 910 270 B1 und US 9 861 770 B2 beschriebene Vakuummotor für den Antrieb eines Lavage-Systems. Der Vakuummotor soll so beschaffen sein, dass das in Operationssälen über zentrale Vakuumanalgen bereit gestellte Vakuum ausreichend für einen Motorbetrieb ist. Der Hub des Vakuummotors sollte größer 5 mm sein, um Werkzeuge wie Bürsten oder Sägen damit im medizinischen Bereich wirkungsvoll betreiben zu können. Der Vakuummotor soll zum Antreiben von Lavage-Systemen und von Vorrichtungen zum Debridement von infiziertem Weich- und Knochengewebe geeignet sein, die aus hygienischen Gründen nur zum einmaligen Gebrauch als sogenannte "disposable Devices" (Wegwerfprodukte) bestimmt sind. Der Vakuummotor soll kostengünstig aus einer möglichst geringen Anzahl von Kunststoffspritzgießteilen mit nur wenigen Montageschritten zu fertigen sein. Der Vakuummotor soll mit Ethylenoxid sterilisierbar sein.

Die der vorliegenden Erfindung zugrundeliegenden Aufgaben werden gelöst durch einen Vakuummotor aufweisend
1) ein Gehäuse mit einem zylindrischen Innenraum, wobei das Gehäuse und der Innenraum jeweils eine Vorderseite und eine der Vorderseite gegenüberliegende Rückseite haben, wobei die Vorderseite des Innenraums der Vorderseite des Gehäuses zugewandt ist,
2) einen Arbeitskolben, der in axialer Richtung der Zylinderachse des zylindrischen Innenraums linear oszillierend beweglich zwischen der Vorderseite und der Rückseite des Innenraums angeordnet ist,
3) ein erstes Rückstellelement, das im Innenraum angeordnet ist und das zumindest zeitweise eine in Richtung der Vorderseite des Innenraums wirkende Kraft auf den Arbeitskolben ausübt,
4) einen Gasauslass im Gehäuse zum Abführen des Gases aus dem Innenraum, wobei der Gasauslass an eine Unterdruckquelle anschließbar oder angeschlossen ist,
5) einen Gaseinlass im Gehäuse zum Einspeisen von Umgebungsluft oder eines unter Druck stehenden Gases in den Innenraum, wobei der Gasauslass und der Gaseinlass zwischen dem Arbeitskolben und der Rückseite des Gehäuses in den Innenraum münden und wobei der Arbeitskolben den Gasauslass und den Gaseinlass in keiner Stellung vollständig abdeckt oder in keiner Stellung auch nur teilweise abdeckt,
6) einen gegen den Gaseinlass beweglichen Ventilkörper zum Verschließen des Gaseinlasses,
7) ein zweites Rückstellelement, das den Ventilkörper in die geschlossene Stellung überführt, so dass der Ventilkörper den Gaseinlass verschließt,
8) wenigstens einen Stößel, der in der axialen Richtung beweglich innerhalb des Gehäuses zwischen dem Arbeitskolben und dem Ventilkörper angeordnet ist, wobei
9) bei einer Bewegung des Arbeitskolbens in Richtung der Rückseite des Innenraums von dem Arbeitskolben über den wenigstens einen Stößel ein Impuls auf den Ventilkörper übertragbar ist, so dass der Ventilkörper gegen die Kraft des zweiten Rückstellelements bewegbar ist und dadurch der Gaseinlass zu öffnen ist und wobei der freie Querschnitt des geöffneten Gaseinlasses zumindest genauso groß ist wie der freie Querschnitt des Gasauslasses.

Der freie Querschnitt des geöffneten Gaseinlasses und des vollständig offenen Gasauslasses ist die als freier Strömungsquerschnitt für die strömenden Gase zur Verfügung stehende Querschnittsfläche an der engsten Stelle. Die freien Querschnitte dienen dem Luftaustausch beziehungsweise Gasaustausch im Innenraum des Vakuummotors.

Unter dem Begriff "Vakuummotor" wird im Rahmen der vorliegenden Erfindung ein Motor verstanden, der mit Hilfe eines Druckgefälles zwischen einem Gas mit Unterdruck und einem Gas mit einem höheren Druck Arbeit leisten kann. Dabei wird als Arbeitsmedium ein Gas bei Luftdruck, Normaldruck oder höher verwendet, besonders bevorzugt Luft aus der Umgebung des Vakuummotors verwendet, und das Gas oder die Luft wird durch den Vakuummotor mit Hilfe des Vakuums beziehungsweise des Unterdrucks geleitet und leistet dabei am Arbeitskolben eine Arbeit. Hierzu ist vorzugsweise vorgesehen, dass auf der der Vorderseite des Innenraums zugewandten Seite des Arbeitskolbens stets der Umgebungsdruck lastet. Hierzu kann vorgesehen sein, dass der vordere Bereich des Innenraums zwischen der Vorderseite des Arbeitskolbens und der Vorderseite des Innenraums mit der Umgebung des Vakuummotors verbunden ist.

Ein Zylinder beziehungsweise eine zylindrische Geometrie im Sinne der vorliegenden Erfindung ist, wie auch nach der allgemeinen Definition, ein von zwei parallelen, ebenen, kongruenten Flächen (Grund- und Deckfläche) und einer Mantelfläche beziehungsweise Zylinderfläche begrenzter Körper, wobei die Mantelfläche von parallelen Geraden gebildet wird. Das heißt, der Zylinder entsteht durch Verschiebung einer ebenen Fläche entlang einer Geraden, die nicht in dieser Ebene liegt. Die Höhe des Zylinders ist gegeben durch den Abstand der beiden Ebenen, in denen Grund- und Deckfläche liegen.

Sind die Geraden senkrecht zu Grund- und Deckfläche, spricht man von einem geraden Zylinder. Die gerade zylindrische Geometrie des Innenraums ist erfindungsgemäß bevorzugt. Ein gerader Kreiszylinder stellt im Sinne der vorliegenden Erfindung also nur einen Spezialfall einer zylindrischen Geometrie dar, ist aufgrund der einfacheren Fertigung aber ebenfalls bevorzugt.

Die Vorderseite und die Rückseite des Gehäuses und des Innenraums sind gleich ausgerichtet.

Damit effizient Arbeit geleistet werden kann, schließt der Arbeitskolben vorzugsweise dicht mit den Innenwänden des zylindrischen Innenraums ab. Der Arbeitskolben ist hierzu dementsprechend vorzugsweise zumindest bereichsweise zylindrisch und passend zum Innenraum. Es kann zumindest eine umlaufende Dichtung am Arbeitskolben vorgesehen sein, die den Arbeitskolben gegen die Innenwand des Innenraums abdichtet.

Es kann vorgesehen sein, dass der Arbeitskolben den Gasauslass in einer Position, in der der Arbeitskolben maximal in Richtung der Rückseite des Innenraums ausgelenkt ist, bereichsweise abdeckt, während der Gaseinlass in keiner Position des Arbeitskolbens im Innenraum abgedeckt ist. Vorzugsweise bleibt der Gasauslass jedoch vollständig geöffnet, das heißt, dass der Arbeitskolben den Gasauslass in keiner Stellung auch nur bereichsweise abdeckt.

Die Masse des Ventilkörpers und die Federkonstante beziehungsweise die elastischen Eigenschaften des zweiten Rückstellelements sind vorzugsweise derart auf die Arbeitsfrequenz des Arbeitskolbens abgestimmt, dass die Eigenschwingung des Ventilkörpers eine zur Oszillation des Arbeitskolbens passende Eigenfrequenz aufweist.

Mit der vorliegenden Erfindung kann bevorzugt vorgesehen sein, dass der Ventilkörper den Gasauslass in keiner Stellung verschließen kann.

Hierdurch wird sichergestellt, dass die Bewegung des Ventilkörpers den Gasauslass nicht beeinträchtigen kann. Zudem muss keine Abdichtung des Gasauslasses erfolgen und der Vakuummotor kann so besonders einfach und kostengünstig aufgebaut werden.

Es kann auch vorgesehen sein, dass ein vorderer Teil des Innenraums über wenigstens eine Belüftungsöffnung im Gehäuse mit der Umgebung des Gehäuses verbunden ist, wobei der vordere Teil des Innenraums in jeder Stellung des Arbeitskolbens durch den Arbeitskolben vom Gasauslass getrennt ist.

Hiermit wird sichergestellt, dass auf der der Vorderseite des Innenraums zugewandten Seite des Arbeitskolbens stets der Umgebungsdruck lastet, so dass der Arbeitskolben von der Druckdifferenz zwischen seiner Vorderseite und einer der Vorderseite des Arbeitskolbens gegenüberliegenden Rückseite gegen die Kraft des ersten Rückstellelements angetrieben werden kann. Damit ist mit dem Vakuummotor eine hohe Leistung erzielbar. Zudem kann der Innenraum so auch im vorderen Teil mit Ethylenoxid sterilisiert werden.

Des Weiteren kann vorgesehen sein, dass das zweite Rückstellelement eine elastische Feder ist, mit der der linear bewegliche Ventilkörper linear federnd gegen den Gaseinlass gelagert ist.

Als elastische Feder kann vorzugsweise eine Spiralfeder verwendet werden.

Elastische Federn sind kostengünstig und für den vorgesehenen Zweck effizient einsetzbar. Die elastische Feder kann aus Metall, wie einem Federstahl, aber auch aus einem elastisch verformbaren Kunststoff bestehen. Wenn der Vakuummotor als hygienisches Wegwerfprodukt konzipiert ist, muss die Feder nicht auf eine lange Lebensdauer ausgelegt sein, so dass auch einfache Kunststoffe verwendet werden können.

Bei besonders bevorzugten Vakuummotoren kann vorgesehen sein, dass der Gaseinlass an der Rückseite des Innenraums angeordnet ist und dass der Gaseinlass ein Ventilelement mit einem zylindrischen Ventilraum und mit einer durch den Ventilkörper verschließbaren Durchführung aufweist, die den Innenraum und den zylindrischen Ventilraum im offenen Zustand verbindet, wobei der Ventilkörper in dem zylindrischen Ventilraum in axialer Richtung des zylindrischen Ventilraums geführt und beweglich gelagert ist und der zylindrische Ventilraum eine Gaseinlassöffnung zum Einspeisen von Umgebungsluft oder eines unter Druck stehenden Gases aufweist. Dabei kann vorzugsweise vorgesehen sein, dass das zweite Rückstellelement eine elastische Feder ist, die zwischen dem Ventilkörper und einer von dem Innenraum abgewandten Rückseite des Ventilraums angeordnet ist.

Hierdurch ist der Ventilkörper im Ventilraum analog dem Arbeitskolben im Innenraum axial beweglich. Der Arbeitskolben kann so den Ventilkörper anstoßen oder anschlagen und dabei aus dem Ventilsitz stoßen oder schlagen und so den Gaseinlass öffnen. Durch die Führung des Ventilkörpers im zylindrischen Ventilraum kann eine stabile Bewegung des Ventilkörpers und ein zuverlässiges und gleichmäßiges Öffnen und Schließen des Gaseinlasses erreicht werden.

Dabei kann auch vorgesehen sein, dass die Durchführung eine kleinere Querschnittsfläche hat als der Ventilraum und die Durchführung an einer dem Innenraum zugewandten Seite in den Ventilraum mündet, so dass der Ventilraum an der dem Innenraum zugewandten Seite einen Ventilsitz bildet und der Ventilkörper in der geschlossenen Stellung mit einer dem Innenraum zugewandten Vorderseite auf dem Ventilsitz aufliegt, wobei in dem Ventilkörper wenigstens ein Durchgang angeordnet ist, der die Vorderseite des Ventilkörpers mit einer der Vorderseite gegenüberliegenden Rückseite des Ventilkörpers verbindet, so dass der wenigstens eine Durchgang und damit der Gaseinlass durch den Ventilsitz verschlossen sind, wenn der Ventilkörper mit dem zweiten Rückstellelement auf den Ventilsitz gedrückt ist.

Hiermit wird erreicht, dass bei einem wegdrücken oder wegschlagen des Ventilkörpers aus dem Ventilsitz schnell der volle freie Querschnitt zum Einströmen von Umgebungsluft oder von Gas zur Verfügung steht.

Bei solchen Vakuummotoren kann auch vorgesehen sein, dass der freie Querschnitt der Durchführung abzüglich der Querschnittsfläche des wenigstens einen Stößels oder aller Stößel mindestens so groß ist wie der freie Querschnitt des Gasauslasses

Hiermit wird sichergestellt, dass der Gasdruck im Innenraum zwischen dem Arbeitskolben und der Rückseite des Innenraums bei geöffnetem Gaseinlass schnell genug steigt, um eine schnelle und kraftvolle Bewegung des Arbeitskolbens in Richtung der Vorderseite des Innenraums zu ermöglichen.

Es kann auch vorgesehen sein, dass das Gehäuse einen vorderen Teil aufweist, der zumindest die Vorderseite des Innenraums begrenzt, und einen hinteren Teil aufweist, der zumindest den Ventilraum begrenzt, wobei der vordere Teil des Gehäuses und der hintere Teil des Gehäuses aneinander befestigt sind, wobei bevorzugt eine von dem Innenraum abgewandte Rückseite des Ventilraums von einem Deckel des hinteren Teils des Gehäuses begrenzt wird, wobei besonders bevorzugt eine Gaseinlassöffnung in dem Deckel angeordnet ist.

Hierdurch wird der Zusammenbau des Vakuummotors vereinfacht.

Ferner kann bei solchen Vakuummotoren vorgesehen sein, dass der Ventilkörper in dem Ventilraum durch Einwirkung des Arbeitskolbens zwischen 0,1 mm und 6 mm bewegbar ist.

Hierdurch kann der Vakuummotor kompakt aufgebaut werden, wobei der Hub bei der Verwendung eines geeigneten Rückstellelements eine für medizinische Zwecke geeignete Arbeitsfrequenz des Motors ergibt.

Gemäß einer alternativen Ausführungsform kann vorgesehen sein, dass der Ventilkörper drehbar gegen den Gaseinlass gelagert ist und das zweite Rückstellelement eine elastische Feder oder Schneckenfeder ist, mit der der drehbar gelagerte Ventilkörper drehbar federnd gegen den Gaseinlass gelagert ist.

Bei dieser Ausführungsform wird ein sich drehender oder ein um eine Drehachse drehbar gelagerter Ventilkörper verwendet, um den Gaseinlass zu öffnen und zu schließen. Dabei wird mit dem zweiten Rückstellelement eine Drehschwingung erzeugt beziehungsweise genutzt, um den Gaseinlass periodisch zu öffnen und zu schließen. Angetrieben wird die Drehschwingung auch hier durch Stöße des Arbeitskolbens. Die vorliegende Erfindung kann also auch mit drehbar gelagerten Ventilkörpern realisiert werden.

Es kann erfindungsgemäß vorgesehen sein, dass eine Stange an einer Vorderseite des Arbeitskolbens angeordnet ist, wobei die Vorderseite des Arbeitskolbens der Vorderseite des Innenraums zugewandt ist und wobei die Stange durch die Vorderseite des Gehäuses hindurch aus dem Gehäuse herausragt und die Stange in einer Führung in der Vorderseite des Gehäuses beweglich gelagert ist.

Hierdurch kann die Leistung des Motors über die Stange von außen nutzbar gemacht werden.

Dabei kann vorgesehen sein, dass an der Vorderseite der Stange ein Befestigungselement, insbesondere ein Gewinde, angeordnet ist, über das ein Werkzeug, wie eine Säge, eine Raspel oder eine Bürste, mit einem zum Befestigungselement passenden Gegenbefestigungselement, insbesondere einem zum Gewinde passenden Gegengewinde, an der Stange befestigbar ist.

Hierdurch ist der Vakuummotor variabel mit verschiedenen Werkzeugen einsetzbar.

Gemäß einer bevorzugten Weiterbildung der vorliegenden Erfindung kann vorgesehen sein, dass der wenigstens eine Stößel an einer der Rückseite des Gehäuses zugewandten Rückseite des Arbeitskolbens oder an einer dem Innenraum zugewandten Vorderseite des Ventilkörpers befestigt ist oder der wenigstens eine Stößel als separates Teil beweglich gegen das Gehäuse gelagert ist, vorzugsweise mit einem dritten Rückstellelement federnd gegen den Arbeitskolben und den Ventilkörper gelagert ist, oder zumindest ein Stößel an einer der Rückseite des Gehäuses zugewandten Rückseite des Arbeitskolbens und zumindest ein weiterer Stößel an einer dem Innenraum zugewandten Vorderseite des Ventilkörpers befestigt ist, wobei bevorzugt der wenigstens eine Stößel oder die zumindest zwei Stößel derart angeordnet ist oder sind, dass bei einer Bewegung des Arbeitskolbens in Richtung der Rückseite des Gehäuses der Ventilkörper über den wenigstens einen Stößel oder über die zumindest zwei Stößel in Richtung der Rückseite des Gehäuses gedrückt oder geschlagen wird.

Wenn als das erste Rückstellelement eine Druckfeder verwendet wird und ein Stößel an der Rückseite des Arbeitskolbens angeordnet ist, ist die Druckfeder vorzugsweise eine Spiralfeder, die den Stößel am Arbeitskolben koaxial umschließt.

Als elastische Druckfeder kann vorzugsweise eine Spiralfeder verwendet werden.

Mit dem wenigstens einen Stößel oder den zumindest zwei Stößeln kann der Arbeitskolben vom Ventilkörper beabstandet gelagert werden. Wenn sowohl am Arbeitskolben als auch am Ventilkörper jeweils ein Stößel angeordnet ist, sind diese vorzugsweise so ausgerichtet, dass sie aufeinandertreffen, um den Ventilkörper zu bewegen beziehungsweise vom Arbeitskolben wegzuschlagen. Hierdurch kann die Verbindung von dem Arbeitskolben zum Ventilkörper kompakt und schmal gestaltet werden und es bleibt (in der Durchführung) genug freier Querschnitt für den Gaseinlass.

Ferner kann vorgesehen sein, dass die Länge des wenigstens einen Stößels oder die Summe der Längen zumindest eines Stößels am Arbeitskolben und zumindest eines weiteren Stößels am Ventilkörper kleiner ist als der Abstand zwischen dem Ventilkörper in der geschlossenen Stellung und der Rückseite des Arbeitskolbens, wenn der Arbeitskolben maximal in Richtung der Vorderseite des Innenraums ausgelenkt ist.

Hiermit wird sichergestellt, dass der Arbeitskolben bei seiner Bewegung in Richtung der Rückseite des Innenraums eine ausreichende Geschwindigkeit aufgenommen hat, wenn er den Ventilkörper mittels des wenigstens einen Stößels oder mittels der zumindest zwei Stößel wegschlägt beziehungsweise wegdrückt. Dadurch kann die Schwingung des Ventilkörpers besser angeregt werden und der Gaseinlass wird schnell vollständig geöffnet. Zudem kann durch den Stoß auch ein Festsaugen des Ventilkörpers verhindert werden.

Zu dem gleichen Zweck kann vorgesehen sein, dass der Arbeitskolben eine größere Masse hat als der Ventilkörper, bevorzugt eine zumindest doppelt so große Masse hat wie der Ventilkörper, besonders bevorzugt eine zumindest zehnmal so große Masse hat wie der Ventilkörper.

Bevorzugt kann auch vorgesehen sein, dass das erste Rückstellelement eine Druckfeder ist, die zwischen einer der Rückseite des Gehäuses zugewandte Rückseite des Arbeitskolbens und der Rückseite des Innenraums angeordnet ist, wobei die Druckfeder den Arbeitskolben in Richtung der Vorderseite des Innenraums drückt.

Die Druckfeder ist die einfachste und eine besondere kostengünstige Ausführung für das erste Rückstelleelement. Die Druckfeder kann dabei aus Metall, insbesondere aus einem Federstahl, bestehen oder auch aus einem elastischen Kunststoff. Wenn der Vakuummotor als hygienisches Wegwerfprodukt konzipiert ist, muss die Druckfeder nicht auf eine lange Lebensdauer ausgelegt sein, so dass auch einfache Kunststoffe verwendet werden können.

Um den Vakuummotor kostengünstig herstellen zu können, leicht desinfizieren zu können und bequem entsorgen zu können, kann vorgesehen sein, dass das Gehäuse, der Arbeitskolben, der Ventilkörper und die Stange aus einem Kunststoff, insbesondere aus einem thermoplastischen Kunststoff gefertigt sind, vorzugsweise durch Spritzgießen geformt sind.

Diese Bauteile können kostengünstig durch Kunststoffspritzgießen hergestellt werden und nach Gebrauch gefahrlos und umweltschonend entsorgt werden. Dabei werden solche Kunststoffe bevorzugt, die eine Sterilisation mit Ethylenoxid tolerieren. Bevorzugt werden, sofern vorhanden, auch der wenigstens eine Stößel und/oder die Stange und besonders bevorzugt auch die Feder(n) beziehungsweise die Druckfeder(n) aus einem Kunststoff, insbesondere aus einem thermoplastischen Kunststoff gefertigt.

Des Weiteren kann vorgesehen sein, dass der Ventilkörper ein rotationssymmetrischer Körper ist, insbesondere ein Zylinder, ein Zylinder mit konischer Spitze oder ogivenförmiger Spitze, eine Kugel oder eine Ogive ist, wobei vorzugsweise die Dreh-Symmetrieachse des rotationssymmetrischen Körpers der Bewegungsrichtung des Ventilkörpers entspricht.

Hierdurch kann die Bewegung des Ventilkörpers gut kontrolliert werden. Zudem kann auch der Schluss des Gaseinlasses bei diesen Formen gut realisiert werden.

Ferner kann vorgesehen sein, dass eine der Rückseite des Innenraums zugewandte Rückseite des Arbeitskolbens einen Durchmesser von mindestens 10 mm hat, bevorzugt einen Durchmesser von mindestens 20 mm, besonders bevorzugt einen Durchmesser von mindestens 30 mm.

Hiermit wird eine für medizinische Anwendungen ausreichende Kraft des Vakuummotors auch bei geringen Druckunterschieden sichergestellt. Beispielsweise kann der Vakuummotor so mit einer Vakuumquelle betrieben werden, die einen Unterdruck von 0,8 bar bereitstellt und durch den Gasauslass im Innenraum erzeugt, während Umgebungsluft (auf Meereshöhe) durch den Gaseinlass eingespeist wird.

Die der vorliegenden Erfindung zugrundeliegenden Aufgaben werden auch gelöst durch ein chirurgisches Antriebssystem aufweisend einen erfindungsgemäßen Vakuummotor sowie ein manuell bedienbares Ventil, wobei das Ventil in einer Leitung, insbesondere in einer Vakuumleitung, angeordnet ist, die mit dem Gasauslass und die mit der Unterdruckquelle verbindbar oder verbunden ist, so dass mit dem Ventil die Verbindung des Gasauslasses zur Unterdruckquelle unterbrechbar und/oder der Unterdruck am Gasauslass einstellbar ist oder das manuell bedienbare Ventil über eine Leitung mit dem Gaseinlass verbunden ist, wobei die Leitung mit der Umgebung oder mit einer Druckgasquelle verbunden ist.

Das Antriebssystem weist die gleichen Vorteile auf, die auch der Vakuummotor aufweist. Der Vakuummotor lässt sich besonders leicht und einfach anhalten, wenn der Gaseinlass mit dem manuell bedienbaren Ventil steuerbar ist. Das manuell bedienbare Ventil ist hierzu vorzugsweise in Strömungsrichtung vor dem Ventilkörper angeordnet.

Dabei kann vorgesehen sein, dass das chirurgische Antriebssystem einen Griff aufweist, mit dem es in einer Hand gehalten werden kann und das Ventil mit einem Abzug am Griff bedienbar ist.

Hierdurch wird das Antriebssystem einfach mit einer Hand einsetzbar.

Des Weiteren kann vorgesehen sein, dass das chirurgische Antriebssystem ein äußeres Gehäuse aufweist, das das Gehäuse des Vakuummotors umschließt und das eine Führung aufweist, durch die die Stange aus dem äußeren Gehäuse herausragt.

Damit kann das Antriebssystem leichter und sicherer bedient werden und der Vakuummotor ist vor äußeren Einflüssen geschützt.

Die der vorliegenden Erfindung zugrundeliegenden Aufgaben werden auch gelöst durch ein medizinisches Lavage-System zum Debridement von Weichgewebe und/oder Knochengewebe aufweisend einen erfindungsgemäßen Vakuummotor oder ein erfindungsgemäßes chirurgisches Antriebssystem.

Die der vorliegenden Erfindung zugrundeliegenden Aufgaben werden auch gelöst durch eine medizinische Vorrichtung zum Bürsten, Raspeln oder Sägen von Weichgewebe und/oder Knochengewebe aufweisend einen erfindungsgemäßen Vakuummotor oder ein erfindungsgemäßes chirurgisches Antriebssystem.

Das chirurgische Antriebssystem, das medizinische Lavage-System oder die medizinische Vorrichtung zum Bürsten, Raspeln oder Sägen von Weichgewebe und/oder Knochengewebe sind als einmalig zu verwendende Vorrichtungen vorgesehen. Dadurch bleibt die jeweilige Vorrichtung hygienisch und kann steril im OP eingesetzt werden.

Die genannten Vorrichtungen weisen die Vorteile auf, die auch der Vakuummotor beziehungsweise das chirurgische Antriebssystem aufweist.

Die der vorliegenden Erfindung zugrundeliegenden Aufgaben werden auch gelöst durch ein Verfahren zum Betreiben eines Vakuummotors, insbesondere eines erfindungsgemäßen Vakuummotors, bei dem ein Arbeitskolben linear in einem zylindrischen Innenraum oszilliert, der Innenraum aufweisend eine Vorderseite und eine der Vorderseite gegenüberliegende Rückseite, wobei das Verfahren die folgenden Schritte umfasst:
A) Evakuieren von Gas aus dem Innenraum zwischen dem Arbeitskolben und der Rückseite des Innenraums durch einen offenen Gasauslass,
B) Bewegen des Arbeitskolbens in Richtung der Rückseite des Innenraums aufgrund des Unterdrucks,
C) Spannen eines ersten Rückstellelements durch die Bewegung des Arbeitskolbens,
D) Öffnen eines Gaseinlasses im Bereich der Rückseite des Innenraums durch die Bewegung des Arbeitskolbens, indem ein Impuls von dem Arbeitskolben über wenigstens einen Stößel auf einen beweglich gelagerten Ventilkörper übertragen wird, wobei der Gaseinlass durch die Bewegung des Ventilkörpers geöffnet wird, wobei der freie Querschnitt des geöffneten Gaseinlasses zumindest genauso groß ist wie der freie Querschnitt des Gasauslasses, wobei zum Öffnen des Gaseinlasses der Ventilkörper gegen die Kraft eines zweiten Rückstellelements bewegt wird und dabei das zweite Rückstellelement gespannt wird, indem während der Bewegung des Arbeitskolbens in Richtung der Rückseite des Innenraums der Arbeitskolben den Ventilkörper mittels des wenigstens einen Stößels am Ventilkörper und/oder am Arbeitskolben anstößt oder anschlägt und aus einem Ventilsitz stößt oder schlägt,
E) Einströmen von Umgebungsluft oder Druckgas in den Innenraum zwischen dem Arbeitskolben und der Rückseite des Innenraums durch den Gaseinlass,
F) Bewegen des Arbeitskolbens in Richtung der Vorderseite des Innenraums angetrieben von dem gespannten ersten Rückstellelement, wobei dabei eine Stange, die an dem Arbeitskolben befestigt ist und durch eine Führung aus der Vorderseite des Innenraums herausgeführt ist, aus dem Innenraum herausgedrückt wird,
G) Verschließen des Gaseinlasses durch eine umgekehrte Bewegung des Ventilkörpers angetrieben durch das Rückstellelement.

Dabei kann vorgesehen sein, dass das Verfahren mit einem erfindungsgemäßen Vakuummotor oder mit einem erfindungsgemäßen chirurgischen Antriebssystem oder mit einem erfindungsgemäßen Lavage-System oder einer erfindungsgemäßen medizinischen Vorrichtung zum Bürsten, Raspeln oder Sägen von Weichgewebe und/oder Knochengewebe durchgeführt wird.

Ferner kann vorgesehen sein, dass der Gasauslass während des gesamten Ablaufs des Verfahrens offen bleibt.

Hierdurch kann auf Maßnahmen zum Schließen des Gasauslasses verzichtet werden. Dadurch kann das Verfahren einfacher, störunanfälliger und kostengünstiger umgesetzt werden.

Durch den Antrieb mit dem zweiten Rückstellelement kann der Ventilkörper eine Schwingung vollführen, bei der der Gaseinlass vollständig geöffnet wird und so eine kraftvolle Bewegung des Arbeitskolbens ermöglicht wird.

Dabei kann vorgesehen sein, dass, nachdem der Ventilkörper von dem Arbeitskolben angestoßen oder angeschlagen wurde, sich der Ventilkörper aufgrund seiner Massenträgheit weiterbewegt und dabei das zweite Rückstellelement weiter gespannt wird.

Dadurch wird der Gaseinlass schnell und vollständig geöffnet.

Bevorzugt kann auch vorgesehen sein, dass der Vakuummotor durch Öffnen des Gaseinlasses oder Gasauslasses angeschaltet und durch Schließen des Gaseinlasses oder Gasauslasses ausgeschaltet wird.

Hierdurch lässt sich der Vakuummotor bequem starten und stoppen. Der Gaseinlass wird dabei nicht mit dem Ventilkörper geöffnet und geschlossen sondern vorzugsweise mit einem manuell bedienbaren Ventil.

Schließlich kann auch vorgesehen sein, dass die Schritte A) bis G) solange wiederholt werden, wie ein ausreichender Unterdruck an dem Gasauslass anliegt oder solange ein Gas durch den Gaseinlass nachströmen kann.

Hierdurch wird eine periodische Bewegung des Arbeitskolbens des Vakuummotors erzeugt.

Der Erfindung liegt die überraschende Erkenntnis zugrunde, dass es durch einen von einem Arbeitskolben zu öffnenden und zu schließenden Gaseinlass bei gleichzeitig immer offenem Gasauslass und durch einen großen freien Querschnitt des geöffneten Gaseinlasses relativ zum freien Querschnitt des Gasauslasses zum Einströmen von Umgebungsluft oder eines komprimierten Gases gelingt, einen denkbar einfachen und dadurch kostengünstigen Vakuummotor beziehungsweise ein Verfahren zu Betreiben eines solchen Vakuummotors bereitzustellen, der weitgehend aus Kunststoff gefertigt werden kann und dadurch als hygienisches Wegwerfprodukt zur Verfügung gestellt werden kann. Gleichzeitig wird durch den einfachen Aufbau, bei dem ein massiver zylindrischer Arbeitskolben in einem hohlzylindrischen Hohlraum bewegt wird, ein laufstabiler Vakuummotor bereitgestellt, der besonders unanfällig für Störungen ist und der gleichmäßig mit stabiler Frequenz läuft. Zudem kann durch den erzeugten Hub eine kraftvolle Bewegung eines Werkzeugs erreicht werden, dass für verschiedene Anwendungen im OP-Bereich einsetzbar ist.

Der Vakuummotor nutzt auch den Effekt, dass es beim konstanten Anlegen eines Vakuums ausreicht, nur den Gaseinlass periodisch zu öffnen und zu schließen, um eine linear oszillierenden Bewegung des Antriebskolbens zu erzeugen. Besonders vorteilhaft kann dabei ein einfacher Ventilkörper zum periodischen Verschluss und zur Öffnung des Gaseinlasses verwendet werden, der so gestaltet ist, dass er durch das Vakuum in den Ventilsitz gesaugt und so zum Verschluss gebracht wird.

Ein beispielhafter erfindungsgemäßer Vakuummotor ist zusammengesetzt aus
a) einem Hohlzylinder,
b) einem Arbeitskolben, der axial im Hohlzylinder beweglich ist,
c) einem hinteren Abschlussteil, das an einer Schmalseite des Hohlzylinders angeordnet ist, wobei das Abschlussteil mindestens eine Durchführung besitzt,
d) einem zylinderförmigen Stößel, der auf einer Stirnseite des Arbeitskolbens so angeordnet ist, dass der Stößel in die Durchführung des Abschlussteils eintauchen kann, wobei der Stößel einen kleineren Querschnitt hat als die Durchführung,
e) mindestens einem Federelement, das sich auf der rückseitigen Stirnseite des Arbeitskolbens und an der Innenwand des Abschlussteils abstützt,
f) mindestens einen Gaseinlass im Hohlzylinder, welcher eine Vakuumquelle mit einem Hohlraum verbindet, der durch die Innenwand des Hohlzylinders, der rückseitigen Stirnseite des Arbeitskolbens mit dem daran angeordneten Stößel und der Innenseite des Abschlussteils gebildet wird,
g) einem Ventilsitz, der als Hohlkörper ausgebildet ist und einen Ventilraum begrenzt, wobei eine Stirnseite des Hohlkörpers mit der Durchführung des Abschlussteils verbunden ist und eine zweite Stirnseite des Ventilsitzes, den Ventilraum des Ventilsitzes mit der umgebenden Atmosphäre gasdurchlässig verbindet,
h) einem Ventilkörper, der im Ventilraum des Ventilsitzes angeordnet ist und axial im Ventilraum beweglich ist, und wobei
i) sich der Stößel axial so lang erstreckt, dass bei Kompression des Federelements der Stößel den Ventilkörper berührt und der Ventilkörper durch Einwirkung des Stößels 0,1 bis 6 mm axial im Ventilraum des Ventilsitzes verschoben werden kann.

Das Abschlussteil (der rückseitige Teil des Gehäuses) begrenzt hier die Rückseite des zylindrischen Innenraums, der durch den Hohlzylinder vorgegeben wird, wobei der Hohlzylinder und das Abschlussteil Teile des Gehäuses im Sinne der vorliegenden Erfindung sind. Der Hohlraum ist der Teil des zylindrischen Innenraums, in dem das Vakuum beziehungsweise der Unterdruck die Arbeit am Arbeitskolben leistet. Der Ventilsitz und der Hohlkörper des Ventilsitzes sind Teile des Gaseinlasses im Sinne der vorliegenden Erfindung. Der Hohlkörper des Ventilsitzes begrenzt den Ventilraum, in dem sich der Ventilkörper bewegt.

Die Querschnittsfläche der Durchführung des Abschlussteils ist dabei größer als die Querschnittsfläche des Gasauslasses. Dadurch kann der Hohlraum ausreichend belüftet werden, so dass das Vakuum im Hohlraum zusammenbrechen kann, obwohl durch den Gasauslass permanent Vakuum gezogen wird. Es ist ferner vorgesehen, dass bei Öffnung des Ventils die Querschnittsfläche der Ventilöffnung (des geöffneten Gaseinlasses) zum Luftaustausch größer ist als die Querschnittsfläche des Gasauslasses.

Erfindungsgemäß kann vorgesehen sein, dass die dem Stößel gegenüberliegende Seite des Arbeitskolbens mindestens eine Aufnahme zum Anschließen von Werkzeugen besitzt. Als Werkzeuge können Bürsten, Raspeln, Sägeblätter angeschlossen werden. Unter dem Begriff Werkzeug wird auch ein Lavage-Vorsatz verstanden. Diese Lavage-Vorsätze besitzen eine Gummimembran, die oszillierend, linear bewegt werden kann. Bei der Bewegung der Membran entsteht ein periodischer Unterdruck gefolgt von einem Überdruck. Diese Druckschwankungen werden zum Pumpen von Spülflüssigkeit (auch Lavagierflüssigkeit genannt), zum Beispiel physiologische Kochsalzlösung oder Ringer-Lactat-Lösung, in den Lavage-Vorsätzen genutzt. Es entsteht ein gepulster Strahl der, der aus der Lavage-Vorrichtung ausgestoßen wird.

Der Ventilkörper ist vorzugsweise hinsichtlich seiner Längsachse rotationssymmetrisch und ist bevorzugt als Zylinder, als Zylinder mit konischer Spitze, als Zylinder mit ogivenförmiger Spitze, als Kugel oder als Ogive ausgebildet. Passend dazu ist vorzugsweise auch der Ventilsitz zur Aufnahme des Ventilkörpers rotationssymmetrisch hinsichtlich seiner Längsachse geformt.

Erfindungsgemäß ist bevorzugt vorgesehen, dass das Federelement als Spiralfeder ausgebildet ist, wobei die Spiralfeder den Stößel koaxial umschließt.

Der Durchmesser des Arbeitskolbens größer 1 cm ist, bevorzugt größer 2 cm und ganz besonders bevorzugt größer 3 cm. Mit steigendem Querschnitt des Arbeitskolbens wird die durch das Vakuum einwirkende Kraft größer.

Es ist erfindungsgemäß bevorzugt, dass der Hohlzylinder, der Arbeitskolben, der Stößel, der der Ventilsitz und der Ventilkörper aus Kunststoff gefertigt sind und ganz besonders bevorzugt aus thermoplastischem Kunststoff. Diese Bauteile können kostengünstig durch Kunststoffspritzgießen hergestellt werden. Dabei werden solche Kunststoffe bevorzugt, die eine Sterilisation mit Ethylenoxid tolerieren.

Der erfindungsgemäße Vakuummotor kann in einfacher Weise durch Öffnen und Verschließen der Zuluft angeschaltet und abgeschaltet werden.

Vorteilhaft kann auch ein Getriebe oder Gestänge mit dem Arbeitskolben verbunden sein. Dadurch ist es möglich, auch rotierende Bewegungen zu erzeugen.

Ein beispielhaftes erfindungsgemäßes Verfahren zur Erzeugung von oszillierenden linearen Bewegungen mit dem beispielhaften erfindungsgemäßen Vakuummotor kann beispielsweise durch die folgenden teilweise nacheinander ablaufenden Schritte gekennzeichnet sein:
a) Entfernen der Luft aus dem Hohlraum des Vakuummotors, durch Anlegen von Vakuum oder Unterdruck an den Gasauslass,
b) Ansaugen des Ventilkörpers in den Ventilsitz und Verschluss der Durchführung des Abschlussteils, und gleichzeitiges Ansaugen des Arbeitskolbens in Richtung des Abschlussteils,
c) Spannen des Federelements durch Bewegung des Arbeitskolbens in Richtung des Abschlussteils,
d) Bei weiterer Bewegung des Arbeitskolbens in Richtung des Abschlussteils, Anstoßen des Stößels an den Ventilkörper,
e) Herausstoßen des Ventilkörpers aus dem Ventilsitz,
f) Weitere axiale Bewegung des Ventilkörpers aus dem Ventilsitz infolge der Massenträgheit des Ventilkörpers,
g) Einströmen von Luft durch den geöffneten Gaseinlass in den Hohlraum des Holzylinders und Abbau des Vakuums beziehungsweise Unterdrucks,
h) Entspannung des Federelements, wobei der Arbeitskolben durch das sich entspannende Federelement entgegengesetzt zum dem Abschlussteil bewegt wird, und
i) Wiederholung der Schritte a) bis h) solange Vakuum an dem Gasauslass anliegt.

Der erfindungsgemäße Vakuummotor wird vorzugsweise als Antrieb von Lavage-Systemen und von Vorrichtungen zum Debridement von Weichgewebe und Knochengewebe verwendet. Der Vakuummotor wird als Antrieb von Vorrichtungen zum Bürsten, Raspeln und Sägen von Weichgewebe und Knochengewebe verwendet. Weiterhin wird der Vakuummotor zum Antrieb von einmalig zu verwendenden medizinischen Vorrichtungen verwendet.

Im Folgenden werden weitere Ausführungsbeispiele der Erfindung anhand von zwölf schematisch dargestellten Figuren erläutert, ohne jedoch dabei die Erfindung zu beschränken. Dabei zeigt:
Figur 1: eine schematische perspektivische Teilschnittansicht eines beispielhaften Vakuummotors im entspannten Anfangszustand bei geöffnetem Gehäuse;
Figur 2: eine schematische perspektivische Teilschnittansicht des Vakuummotors nach Figur 1 im gespannten Zustand bei geöffnetem Gehäuse;
Figur 3: eine schematische perspektivische Teilschnittansicht des Vakuummotors im entspannten Zustand bei geöffnetem Gehäuse aus einer anderen Perspektive;
Figur 4: eine schematische Querschnittansicht durch den Vakuummotor nach den Figuren 1 bis 4 im gespannten Zustand;
Figur 5: eine schematische Querschnittansicht durch den Vakuummotor nach den Figuren 1 bis 5 im entspannten Zustand;
Figur 6: eine schematische perspektivische Außenansicht des erfindungsgemäßen Vakuummotors;
Figur 7: eine schematische perspektivische Teilschnittansicht eines erfindungsgemäßen chirurgischen Antriebssystems bei geöffnetem Außengehäuse, das einen Vakuummotor nach den Figuren 1 bis 6 enthält;
Figur 8: eine schematische Querschnittansicht des chirurgischen Antriebssystems nach Figur 7;
Figur 9: eine schematische Querschnittansicht durch den Griff und das Ventil des chirurgischen Antriebssystems;
Figur 10: eine schematische perspektivische Außenansicht des chirurgischen Antriebssystems;
Figur 11: eine schematische perspektivische Außenansicht des chirurgischen Antriebssystems mit einer Bürste als Werkzeug; und
Figur 12: eine schematische perspektivische Teilquerschnittansicht des chirurgischen Antriebssystems bei geöffnetem äußerem Gehäuse mit einer Raspel als Werkzeug.

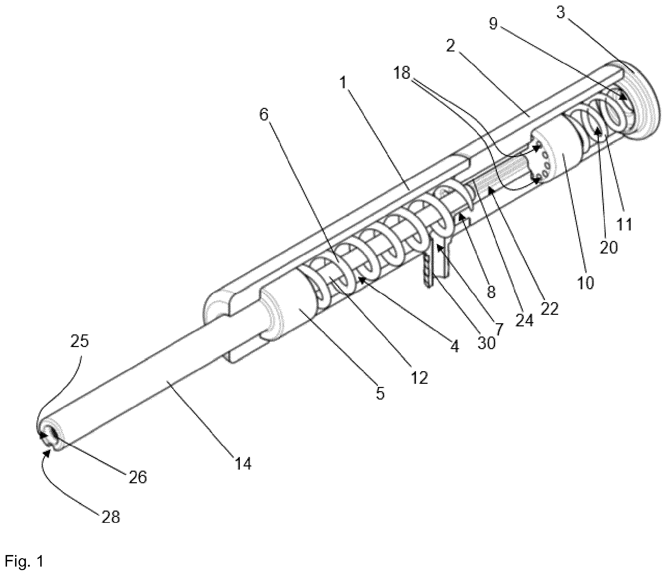

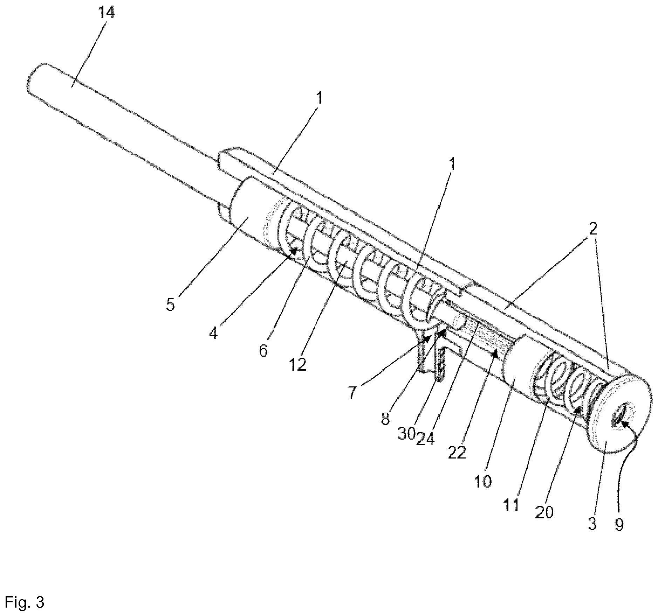

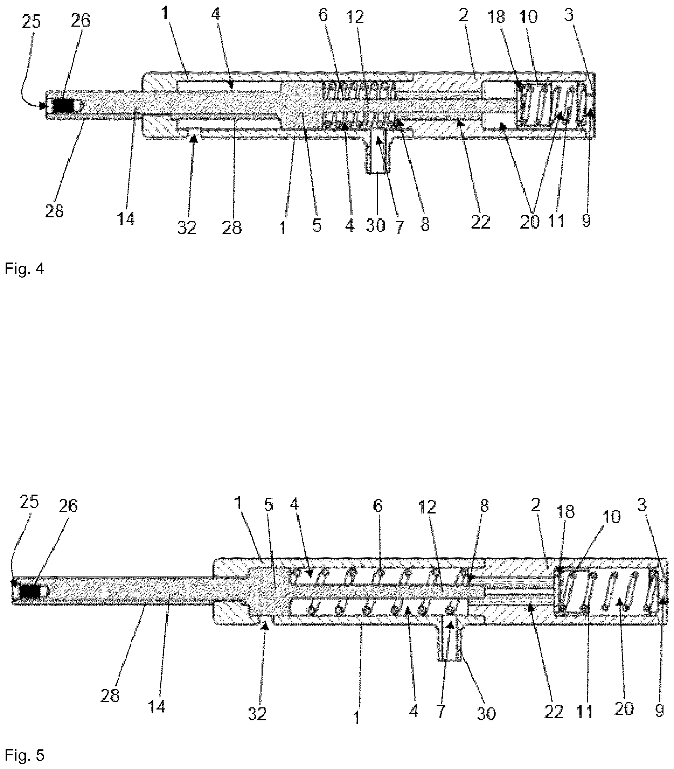

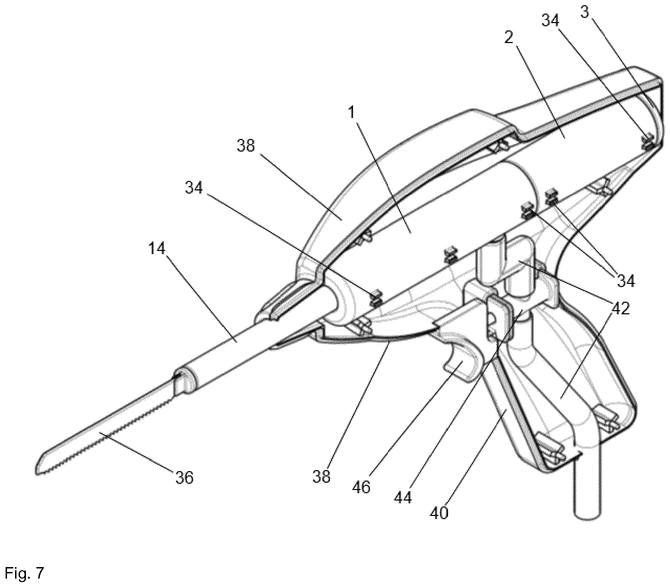

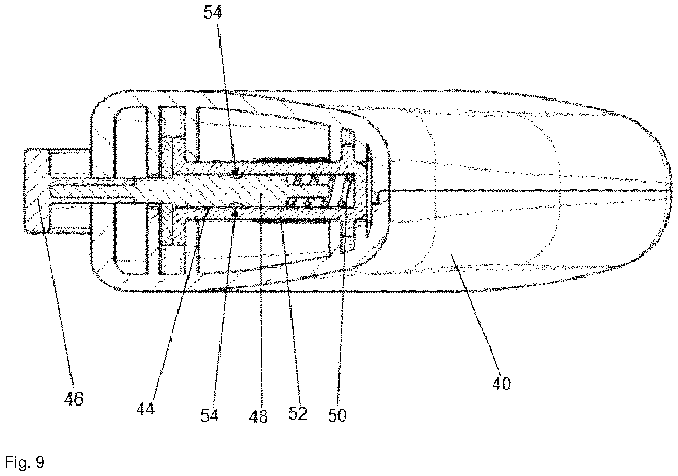

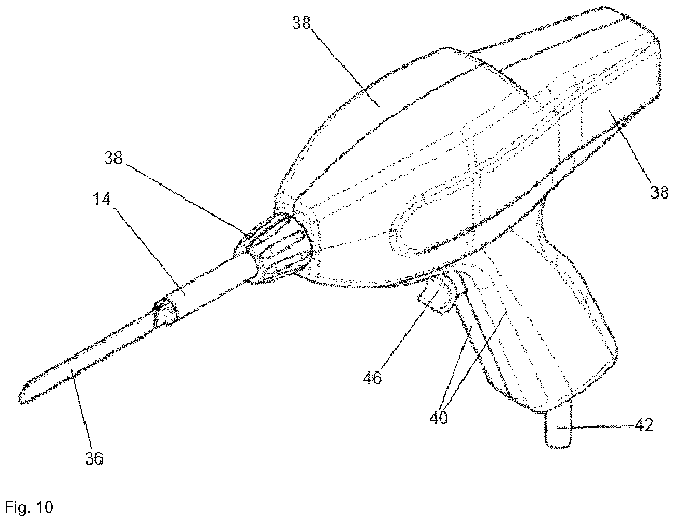

In den Figuren 1 bis 6 sind Abbildungen eines erfindungsgemäßen Vakuummotors gezeigt. Die Figuren 7 bis 10 zeigen ein erfindungsgemäßes chirurgisches Antriebssystem mit einem solchen Vakuummotor, wie er in den Figuren 1 bis 6 gezeigt ist. Die Vorderseite des Vakuummotors und des chirurgischen Antriebssystems und damit die gleichartig ausgerichteten Vorderseiten der Teile des Vakuummotors und des chirurgischen Antriebssystems sind in den Figuren 1, 2, 6, 7 und 10 bis 12 links vorne (aus der Bildebene in Richtung Betrachter hinaus), in Figur 3 links hinten (in die Bildebene hinein, vom Betrachter weg), in den Figuren 4, 5 und 8 links und in Figur 9 auch links, wobei das vordere Ende in Figur 9 nicht gezeigt ist. Grundsätzlich ist die Vorderseite in den Abbildungen also in Richtung der linken Seite ausgerichtet. Während man in den Figuren 1, 2, 6, 7 und 10 bis 12 auch auf die Vorderseiten das Vakuummotors und des chirurgischen Antriebssystems sieht, sieht man in Figur 3 auf die Rückseite des Vakuummotors.

Der Vakuummotor hat ein dreiteiliges Gehäuse 1, 2 aus Kunststoff mit einem vorderen Teil 1 und einem hinteren Teil 2, der auf der Rückseite einen Deckel 3 aufweist. Im Inneren des Gehäuses 1, 2 ist ein zylindrischer Innenraum 4, der an seiner Vorderseite (links) und an den seitlichen Zylindermantelflächen durch das vordere Teil 1 begrenzt ist und der an seiner Rückseite durch den hinteren Teil 2 begrenzt ist. Im Innenraum 4 ist ein zylindrischer Arbeitskolben 5 aus Kunststoff angeordnet, der bezüglich der Zylinderachse des zylindrischen Innenraums 4 und des Arbeitskolbens 5 selbst in beide axialen Richtungen beweglich gelagert ist. Dadurch kann der Arbeitskolben 5 im Innenraum 4 oszillieren. Der Arbeitskolben 5 trennt den Innenraum 4 dicht in einen vorderen Teil und einen hinteren Teil des Innenraums 4. Eine vollständige gasdichte Trennung ist für den Betrieb des Vakuummotors nicht zwingen notwendig, solange der Arbeitskolben 5 nur so dicht gegen die Wände des Innenraums 4 abdichtet, dass eine Druckdifferenz zwischen dem hinteren Teil des Innenraums 4 und dem vorderen Teil des Innenraums 4 erzeugbar ist, mit dem der Arbeitskolben 5 ausreichend weit gegen eine erste Feder 6 bewegt werden kann.

Die erste Feder 6 ist hierzu zwischen dem Arbeitskolben 5 und dem rückseitigen Ende des Innenraums 4 als erstes Rückstellelement für den Arbeitskolben 5 im Innenraum 4 (genauer im hinteren Teil des Innenraums 4) angeordnet. Die erste Feder 6 drückt den Arbeitskolben 5 in Richtung der Vorderseite des Innenraums 4. Ein Gasauslass 7 zum Evakuieren von Gas aus dem Innenraum 4 mündet in den hinteren Teil des Innenraums 4 zwischen dem Arbeitskolben 5 und dem rückseitigen Ende des Innenraums 4. Am rückseitigen Ende des Innenraums 4 mündet auch ein Gaseinlass 8 in den Innenraum 4. Der Gaseinlass 8 reicht im hinteren Teil 2 des Gehäuses 1, 2 bis zum Deckel 3 und ist über eine Gaseinlassöffnung 9 mit der Umgebung des Vakuummotors gasdurchlässig verbunden. Anstelle der Druckfeder 6 im hinteren Teil des Innenraums 4 oder zusätzlich dazu kann auch eine Zugfeder (nicht gezeigt) im vorderen Teil des Innenraums 4 angeordnet und an der Vorderseite des Arbeitskolbens 5 und an der Vorderseite des Innenraums 4 befestigt sein, wobei die Zugfeder den Arbeitskolben 5 in Richtung der Vorderseite des Innenraums 4 zieht.

Zur Unterbrechung der Luftzufuhr beziehungsweise Gaszufuhr in den Innenraum 4 ist im Gaseinlass 8 ein federnd gelagertes Ventil mit einem becherartigen Ventilkörper 10 mit zylindrischer Außenform und einer geschlossenen Vorderseite angeordnet, der von einer zweiten Feder 11 in Richtung einer Abstufung als Ventilsitz gedrückt wird. Die zweite Feder 11 ist das zweite Rückstellelement des Vakuummotors. Die zweite Feder 11 reicht bis in die geöffnete Rückseite des Ventilkörpers 10.

An der Rückseite des Arbeitskolbens 5 ist ein Stößel 12 befestigt, mit dem der Ventilkörper 10 bei einer Bewegung des Arbeitskolbens 5 in Richtung der Rückseite des Innenraums 4 aus dem Ventilsitzt gedrückt, gestoßen beziehungsweise nach hinten geschlagen werden kann. An der Vorderseite des Arbeitskolbens 5 ist eine Stange 14 zur Befestigung eines Werkzeugs 36, 56, 58 befestigt, das aus dem vorderen Teil 1 des Gehäuses 1, 2 ragt und in dem vorderen Teil 1 in axialer Richtung beweglich gelagert ist. Anstelle nur eines einzigen Stößels 12 am Arbeitskolben 5 kann alternativ auch ein Stößel an der Vorderseite des Ventilkörpers 10 angeordnet sein oder es können sowohl am Arbeitskolben 5 als auch am Ventilkörper 10 Stößel vorgesehen sein, die bei einer Bewegung des Arbeitskolbens 5 in Richtung der Rückseite des Innenraums 4 aufeinandertreffen, so dass der Ventilkörper 10 bewegt wird. Theoretisch können auch mehrere Stößel nebeneinander oder ineinander laufen. Der Stößel 12 dient der Impulsübertragung vom Arbeitskolben 5 auf den Ventilkörper 10 bei einer Bewegung des Arbeitskolbens in Richtung der Rückseite des Gehäuses 1, 2.

Alternativ wäre auch vorstellbar, dass der Ventilkörper 10 und der Arbeitskolben 5 über ein umgelenktes Kabel oder Seil (nicht gezeigt) miteinander verbunden sind, wobei das Kabel oder das Seil an der Vorderseite des Arbeitskolbens 5 und an der Rückseite des Ventilkörpers 10 angeordnet sind oder bei umgekehrt angeordnetem Ventilsitz des Ventilkörpers 10 auch an der Vorderseite des Arbeitskolbens 5 und an der Vorderseite des Ventilkörpers 10. Das Kabel oder Seil ist dabei vorzugsweise nicht straff gespannt, wenn der Arbeitskolben 5 maximal in Richtung der Vorderseite des Innenraums 4 gedrückt ist (analog Figur 1). Diese Alternative ist jedoch aufwendiger im Aufbau. Gemäß einer weiteren möglichen alternativen Ausführung kann der Arbeitskolben 5 seine Kraft auch über eine magnetische Kopplung mit dem Ventilkörper 10 gekoppelt sein. Magnete sind jedoch ebenfalls nachteilig und erhöhen die Kosten des Vakuummotors. Weitere Kopplungen zur Übertragung von Kraft beziehungsweise Impuls von der Bewegung des Arbeitskolbens auf den Ventilkörper sind vorstellbar.

In der geschlossenen Vorderseite des Ventilkörpers 10 sind zwölf durchgehende Bohrungen 18 als Gasleitungen vorgesehen. Die Bohrungen 18 verbinden die Vorderseite mit der Rückseite des Ventilkörpers 10. Der Ventilkörper 10 liegt außen an den Innenwänden eines zylindrischen Ventilraums 20 an und trennt diesen in zwei Teile, die durch die Bohrungen 18 miteinander verbunden sind. Wenn der Ventilkörper 10 mit seiner Vorderseite an der Abstufung an der Vorderseite des Ventilraums 20 anliegt, verdeckt der so gebildete Ventilsitz die Bohrungen 18, so dass das Ventil geschlossen ist und keine Luft beziehungsweise kein Gas mehr durch den Gaseinlass 8 in den Innenraum 4 strömen kann. Der hintere Teil des Innenraums 4 kann dann durch den Gasauslass 7 evakuiert werden. Wenn der Ventilkörper 10 mit dem Stößel 12 gegen die Kraft der zweiten Feder 11 in Richtung der Rückseite des Ventilraums 20 aus dem Ventilsitz herausgedrückt ist, liegen die Bohrungen 18 offen und Luft beziehungsweise Gas kann durch den Gaseinlass 8 in den Innenraum 4 nachströmen.

Dazu ist der Ventilraum 20 mit dem Innenraum 4 über eine Durchführung 22 verbunden. Die Durchführung 22 ist im hinteren Teil 2 des Gehäuses 1, 2 ausgeformt. Der Stößel 12 ist in der Durchführung 22 in axialer Richtung beweglich gelagert. Hierzu sind Leisten 24 an der Innenwand der Durchführung 22 vorgesehen, die in axialer Richtung (von hinten nach vorne) ausgerichtet sind und die den Stößel 12 in axialer Richtung führen. Die freien Zwischenräume zwischen der Innenwand der Durchführung 22 und dem Stößel 12, die Summe der freien Querschnittsflächen der Bohrungen 18, der Ventilraum 20 und die Gaseinlassöffnung 9 haben einen freien Querschnitt der zumindest genausgroß oder größer ist als der freie Querschnitt des Gasauslasses 7, beziehungsweise als der freie Querschnitt eines geöffneten Ventils 44 (siehe Figuren 7 bis 9 und 12). Hierdurch wird sichergestellt, dass durch den Gaseinlass 8 mehr Luft beziehungsweise Gas nachströmen kann, als durch den Gasauslass 9 abgesaugt werden kann. Dadurch kann die erste Feder 6 den Arbeitskolben 5 wieder zur Vorderseite des Innenraums 4 drücken.

In der Vorderseite des Stange 14 ist eine Vertiefung 25 mit einem Innengewinde 26 angeordnet, in die die Werkzeuge 36, 56, 58 geschraubt werden können, so dass die Werkzeuge 36, 56, 58 an der Stange 14 befestigt sind. An der Unterseite der Stange 14 (in den Figuren 1 bis 8 und 10 bis 12 unten) ist eine sich in Längsrichtung erstreckende Nut 28 vorgesehen. Die dient der stabilen und linearen Führung der Stange 14. Zur Lagerung weist der vordere Teil 1 des Gehäuses 1, 2 einen Vorsprung auf, der in die Nut 28 greift und in der Nut 28 wie in einer Schiene läuft, wenn der Arbeitskolben 5 oszilliert.

An der Unterseite des vorderen Teils 1 des Gehäuses 1, 2 ist ein Stutzen 30 zur Befestigung eines Schlauchs oder einer Vakuumleitung 42 angeordnet. Der Gasauslass 7 ist im Stutzen 30 angeordnet, so dass der Gasauslass 7 über den Stutzen 30 mit einer Vakuumquelle verbunden werden kann, beziehungsweise verbunden ist.

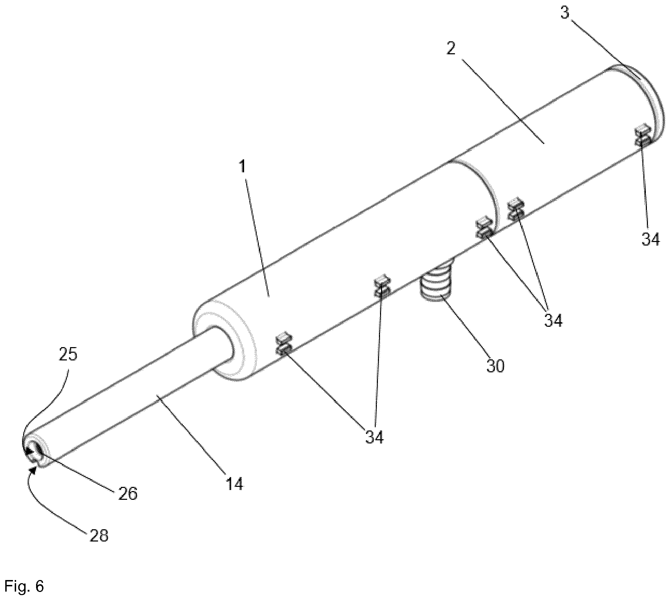

Der vordere Teil des Innenraums 4 ist über eine Belüftungsöffnung 32 mit der Umgebung des Vakuummotors verbunden. Dadurch kann durch die Belüftungsöffnung 32 Luft aus der Umgebung nachströmen, wenn der Arbeitskolben 5 in Richtung der Rückseite des Innenraums 4 bewegt wird. Auf diese Weise erhält man eine möglichst große Druckdifferenz zwischen der Vorderseite des Arbeitskolbens 5 und der Rückseite des Arbeitskolbens 5. Die Belüftungsöffnung 32 ist dichter an der Vorderseite des Innenraums 4 angeordnet als die Höhe des zylindrischen Arbeitskolbens 5. Dadurch wird vermieden, dass die Belüftungsöffnung 32 mit dem hinteren Teil des Innenraums 4 verbindbar ist. Die Belüftungsöffnung 32 ist also vom hinteren Teil des Innenraums 4 zwischen der Rückseite des Arbeitskolbens 5 und der Rückseite des Innenraums 4 durch den Arbeitskolben 5 getrennt, vorzugsweise gasdicht getrennt. Außen sind am Vakuummotor Abstandhalter 34 (siehe Figur 6) angeordnet, die dem einfacheren Verbauen des Vakuummotors in einem chirurgischen Antriebssystem (siehe Figuren 7 bis 12) dienen.

Der beispielhaft gezeigte Vakuummotor läuft im Betrieb wie folgt: Der Vakuummotor befindet sich zunächst im entspannten Ausgangszustand (siehe Figur 1). Als nächstes wird (beispielsweise durch Öffnen des Ventils 44) ein Vakuum beziehungsweise ein Unterdruck an den Gasauslass 7 angelegt. Luft wird aus dem Innenraum 4 zwischen dem Arbeitskolben 5 und der Rückseite des Innenraums 4 durch den Gasauslass 7 abgesaugt. Da die zweite Feder 11 den Ventilkörper 10 in den Ventilsitz drückt und dadurch die Bohrungen 18 abgedeckt sind, kann keine Luft durch die Durchführung 22 und den Gaseinlass 8 in den hinteren Teil des Innenraums 4 nachströmen. Im hinteren Teil des Innenraums 4 entsteht dadurch ein Unterdruck, der an der Rückseite des Arbeitskolbens 5 anliegt. Der vordere Teil des Innenraums 4 ist durch die Belüftungsöffnung 32 mit der Umgebung verbunden. Dadurch lastet auf der Vorderseite des Arbeitskolbens 5 Normaldruck. Die Druckdifferenz zwischen der Vorderseite des Arbeitskolbens 5 und der Rückseite des Arbeitskolbens 5 bewirkt eine Kraft, die den Arbeitskolben 5 in Richtung der Rückseite des Innenraums 4 beziehungsweise der Rückseite des Gehäuses 1, 2 drückt und bewegt. Dabei strömt Luft aus der Umgebung durch die Belüftungsöffnung 32 in den vorderen Teil des Innenraums 4 nach.

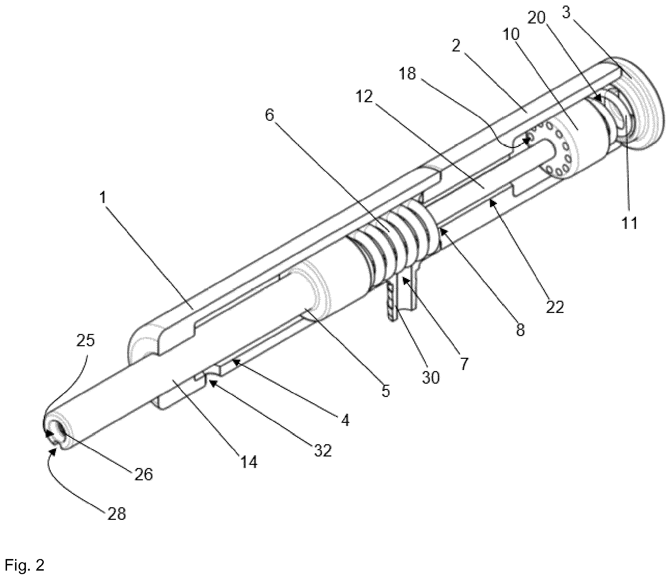

Während der Bewegung des Arbeitskolbens 5 wird die erste Feder 6 gespannt und der Stößel 12 trifft auf den Ventilkörper 10 und schlägt diesen aus dem Ventilsitz. Dabei wird der Ventilkörper 10 gegen die zweite Feder 11 gedrückt und dabei die zweite Feder 11 im Ventilraum 20 gespannt. Sobald der Ventilkörper 10 aus dem Ventilsitz geschlagen ist, sind die Bohrungen 18 nicht mehr abgedeckt und Luft aus der Umgebung kann durch die Gaseinlassöffnung 9, den Ventilraum 20, die Bohrungen 18 und die Durchführung 22 also den Gaseinlass 8 nachströmen. In dieser Situation ist die erste Feder 6 und damit der Vakuummotor gespannt (siehe Figuren 2 und 4). Da die freien Strömungsquerschnitte der Leitungen des Gaseinlasses 8 gleich groß oder größer sind als die freien Querschnitte im oder am Gasauslass 7, strömt zumindest genauso viel Luft nach wie abgesaugt wird. Dadurch bricht der Unterdruck im hinteren Teil des Innenraums 4 zusammen und die durch die Druckdifferenz zwischen der Vorderseite und der Rückseite des Arbeitskolbens 5 wirkende Kraft wird zumindest halbiert. Die erste Feder 6 drückt den Arbeitskolben 5 mit dem Stößel 12 und der Stange 14 wieder nach vorne. Der hintere Teil des Innenraums 4 vergrößert sich und Luft strömt durch den geöffneten Gaseinlass 8 nach. Schließlich schwingt der Ventilkörper 10 angetrieben von der gespannten zweiten Feder 11 wieder zurück und die zweite Feder 11 drückt den Ventilkörper 10 wieder in den Ventilsitz, so dass die Bohrungen 18 und die Durchführung 22 verschlossen werden. Dadurch kann keine Luft mehr durch den nun geschlossenen Gaseinlass 8 mehr in den hinteren Teil des Innenraums 4 nachströmen. Diese Situation, bei der die erste Feder 6 entspannt ist, ist in den Figuren 1, 3 und 5 gezeigt. Dadurch wird der Gasdruck im hinteren Teil des Innenraums 4 wieder durch Absaugen von Luft durch den Gasauslass 7 verringert und die Bewegung beginnt von vorne.

Diese Zyklen wiederholen sich, solange Gas durch den Gasauslass 7 abgesaugt wird. Alternativ kann auch die Luftzufuhr, also der Gaseinlass 8 verschlossen werden, um die Bewegung des Vakuummotors anzuhalten beziehungsweise zu stoppen.

Die Figuren 7 bis 12 zeigen chirurgische Antriebssysteme mit einem erfindungsgemäßen Vakuummotor. Bei dem Antriebssystem nach den Figuren 7 bis 10 ist eine Säge 36 als Werkzeug an der Stange 14 befestigt, so dass das Antriebssystem zum Sägen verwendet werden kann.

Das Antriebssystem hat ein äußeres Gehäuse 38 aus Kunststoff, mit dem der Vakuummotor im Inneren umbaut ist. Lediglich an der Vorderseite des äußeren Gehäuses 38 ist eine Führung für die Stange 14 angeordnet, so dass die Stange 14 mit der Säge 36 daran oder mit einer Bürste 56 oder einer Raspel 58 daran genutzt werden kann. Der Vakuummotor sitzt mit den Abstandhaltern 34 stabil in dem äußeren Gehäuse 38.

Die periodische lineare Oszillation der Stange 14 und der Säge 36 beziehungsweise dem Werkzeug 36, 56, 58 kann genutzt werden, um chirurgische Arbeiten auszuführen. Zur einfacheren Bedienung des chirurgischen Antriebssystems ist das Gehäuse 38 an seiner Unterseite als Griff 40 ausgebildet. An den Stutzen 30 ist eine Vakuumleitung 42 angeschlossen. In der Vakuumleitung 42 ist ein von außen bedienbares Ventil 44 zur Steuerung des Gasflusses aus dem Innenraum 4 und durch den Gasauslass 7 und die Vakuumleitung 44 angeordnet. Das Ventil 44 lässt sich mit einem Abzug 46 am Griff 40 bedienen. Dadurch kann das chirurgische Antriebssystem in einer Hand gehalten und mit dieser Hand bedient werden.

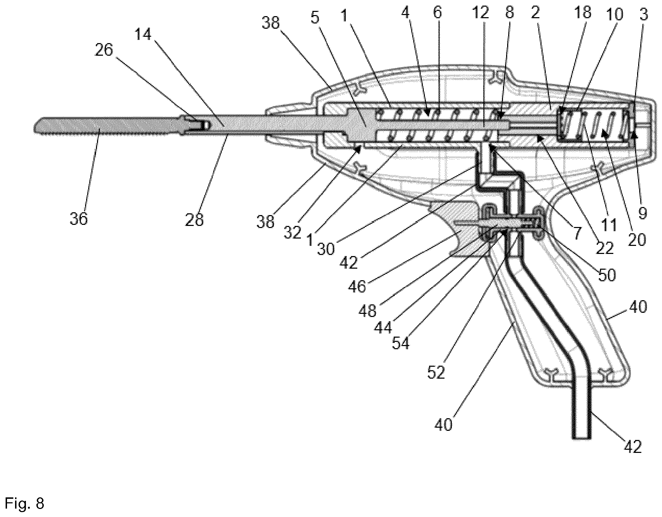

Der Innere Aufbau des Ventils 44 ist in den Querschnittdarstellungen der Figuren 8 und 9 erkennbar. Das Ventil 44 hat einen Ventilstift 48, der gegen eine dritte Feder 50 linear beweglich in einer Hülse 52 gelagert ist. Senkrecht zu der Hülse 52 verläuft ein Rohrstück, das einen Teil der Vakuumleitung 42 bildet und das mit der Hülse 52 eine Kreuzung bildet. Der Ventilstift 48 verschließt das Rohrstück vollständig. In dem ansonsten bereichsweise zylindrischen Ventilstift 48 ist eine umlaufende Ringnut 54 vorgesehen.

Wird der Ventilstift 48 mit Hilfe des Abzugs 46 mit der Ringnut 54 in das Rohrstück gedrückt, das die Teile der Vakuumleitung 42 verbindet, kann Luft durch den Zwischenraum zwischen dem Rohrstück und der Ringnut 54 strömen. Das Ventil 44 ist dann geöffnet. Der freie Strömungsquerschnitt des Ventils 44 entspricht dann der doppelten Querschnittsfläche der Ringnut und ist kleiner als die Summe der Querschnittsflächen der Bohrungen 18 und damit kleiner als der freie Strömungsquerschnitt des geöffneten Ventils des Gaseinlasses 8.

Die Arbeitsweise des chirurgischen Antriebssystems entspricht der des Vakuummotors, wobei die Gasabfuhr aus dem hinteren Teil des Innenraums 4 durch den Gasauslass 7 mit Hilfe des Ventils 44 manuell gesteuert wird.

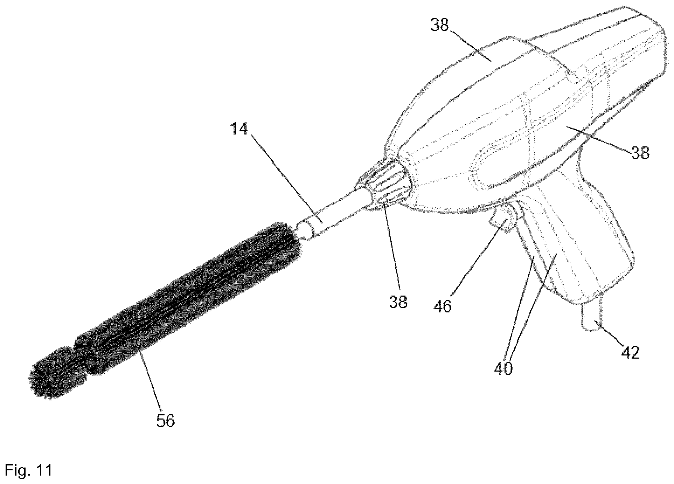

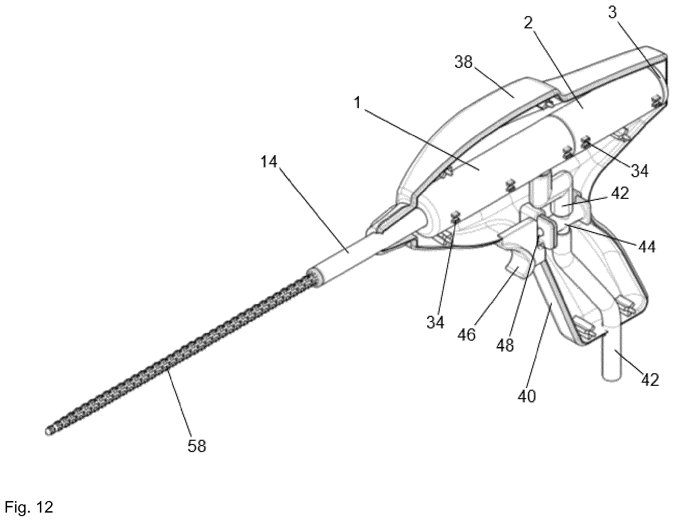

Figur 11 zeigt eine schematische perspektivische Außenansicht des chirurgischen Antriebssystems, wobei eine Bürste 56 anstelle der Säge 36 als alternatives Werkzeug an der Stange 14 des Vakuummotors befestigt ist. Figur 12 zeigt eine schematische perspektivische Teilquerschnittansicht des chirurgischen Antriebssystems bei geöffnetem äußeren Gehäuse 38, wobei eine Raspel 58 anstelle der Säge 36 als alternatives Werkzeug an der Stange 14 des Vakuummotors befestigt ist.

Alternativ zu den Werkzeugen 36, 56, 58 kann auch eine Flüssigkeitspumpe an die Stange 14 angeschlossen und mit dem Vakuummotor betrieben werden, so dass mit dem Vakuummotor bei jedem Bewegungszyklus des Arbeitskolbens ein Sprühstoß mit einer medizinischen Spülflüssigkeit erzeugt wird. Auf diese Weise lässt sich ein erfindungsgemäßes Lavage-System realisieren.
- 1: Gehäuse (vorderes Teil)
- 2: Gehäuse (rückseitiges Teil)
- 3: rückseitiger Deckel des Gehäuses
- 4: Innenraum
- 5: Arbeitskolben
- 6: Feder
- 7: Gasauslass
- 8: Gaseinlass (Einmündung in den Innenraum)
- 9: Gaseinlassöffnung des Gaseinlasses
- 10: Ventilkörper
- 11: Feder
- 12: Stößel
- 14: Stange
- 18: Gasleitung
- 20: Ventilraum
- 22: Durchführung
- 24: Leiste
- 25: Vertiefung
- 26: Innengewinde
- 28: Nut
- 30: Stutzen
- 32: Belüftungsöffnung
- 34: Abstandhalter
- 36: Säge / Werkzeug
- 38: Äußeres Gehäuse
- 40: Griff
- 42: Vakuumleitung
- 44: Ventil
- 46: Abzug / Bedienelement
- 48: Ventilstift
- 50: Feder
- 52: Hülse
- 54: Ringnut
- 56: Bürste
- 58: Raspel

## Patentansprüche

1. Vakuummotor aufweisend
ein Gehäuse (1, 2) mit einem zylindrischen Innenraum (4), wobei das Gehäuse (1, 2) und der Innenraum (4) jeweils eine Vorderseite und eine der Vorderseite gegenüberliegende Rückseite haben, wobei die Vorderseite des Innenraums (4) der Vorderseite des Gehäuses (1, 2) zugewandt ist,
einen Arbeitskolben (5), der in axialer Richtung der Zylinderachse des zylindrischen Innenraums (4) linear oszillierend beweglich zwischen der Vorderseite und der Rückseite des Innenraums (4) angeordnet ist,
ein erstes Rückstellelement (6), das im Innenraum (4) angeordnet ist und das zumindest zeitweise eine in Richtung der Vorderseite des Innenraums (4) wirkende Kraft auf den Arbeitskolben (5) ausübt,
einen Gasauslass (7) im Gehäuse (1, 2) zum Abführen des Gases aus dem Innenraum (4), wobei der Gasauslass (7) an eine Unterdruckquelle anschließbar oder angeschlossen ist,
einen Gaseinlass (8) im Gehäuse (1, 2) zum Einspeisen von Umgebungsluft oder eines unter Druck stehenden Gases in den Innenraum (4), wobei der Gasauslass (7) und der Gaseinlass (8) zwischen dem Arbeitskolben (5) und der Rückseite des Gehäuses (1, 2) in den Innenraum (4) münden und wobei der Arbeitskolben (5) den Gasauslass (7) und den Gaseinlass (8) in keiner Stellung vollständig abdeckt oder in keiner Stellung auch nur teilweise abdeckt,
einen gegen den Gaseinlass (8) beweglichen Ventilkörper (10) zum Verschließen des Gaseinlasses (8),
wenigstens einen Stößel (12), der in der axialen Richtung beweglich innerhalb des Gehäuses (1, 2) zwischen dem Arbeitskolben (5) und dem Ventilkörper (10) angeordnet ist,
**gekennzeichnet durch**
ein zweites Rückstellelement (11), das den Ventilkörper (10) in die geschlossene Stellung überführt, so dass der Ventilkörper (10) den Gaseinlass (8) verschließt, wobei bei einer Bewegung des Arbeitskolbens (5) in Richtung der Rückseite des Innenraums (4) von dem Arbeitskolben (5) über den wenigstens einen Stößel (12) ein Impuls auf den Ventilkörper (10) übertragen wird, so dass der Ventilkörper (10) gegen die Kraft des zweiten Rückstellelements (11) bewegt wird und dadurch der Gaseinlass (8) geöffnet wird, und wobei der freie Querschnitt des geöffneten Gaseinlasses (8) zumindest genauso groß ist wie der freie Querschnitt des Gasauslasses (7).

2. Vakuummotor nach Anspruch 1, **dadurch gekennzeichnet, dass** der Ventilkörper (10) den Gasauslass (7) in keiner Stellung verschließen kann.

3. Vakuummotor nach Anspruch 1 oder 2, **dadurch gekennzeichnet, dass** ein vorderer Teil des Innenraums (4) über wenigstens eine Belüftungsöffnung (32) im Gehäuse (1, 2) mit der Umgebung des Gehäuses (1, 2) verbunden ist, wobei der vordere Teil des Innenraums (4) in jeder Stellung des Arbeitskolbens (5) durch den Arbeitskolben (5) vom Gasauslass (7) getrennt ist.

4. Vakuummotor nach einem der Ansprüche 1 bis 3, **dadurch gekennzeichnet, dass** das zweite Rückstellelement (11) eine elastische Feder (11) ist, mit der der linear bewegliche Ventilkörper (10) linear federnd gegen den Gaseinlass (8) gelagert ist.

5. Vakuummotor nach einem der vorangehenden Ansprüche, **dadurch gekennzeichnet, dass**
der Gaseinlass (8) an der Rückseite des Innenraums (4) angeordnet ist und dass der Gaseinlass (8) ein Ventilelement mit einem zylindrischen Ventilraum (20) und mit einer durch den Ventilkörper (10) verschließbaren Durchführung (22) aufweist, die den Innenraum (4) und den zylindrischen Ventilraum (20) im offenen Zustand verbindet, wobei der Ventilkörper (10) in dem zylindrischen Ventilraum (20) in axialer Richtung des zylindrischen Ventilraums (20) geführt und beweglich gelagert ist und der zylindrische Ventilraum (20) eine Gaseinlassöffnung (9) zum Einspeisen von Umgebungsluft oder eines unter Druck stehenden Gases aufweist, wobei vorzugsweise das zweite Rückstellelement (11) eine elastische Feder (11) ist, die zwischen dem Ventilkörper (10) und einer von dem Innenraum (4) abgewandten Rückseite des Ventilraums (20) angeordnet ist.

6. Vakuummotor nach einem der Ansprüche 1 bis 3, **dadurch gekennzeichnet, dass** der Ventilkörper drehbar gegen den Gaseinlass (8) gelagert ist und das zweite Rückstellelement eine elastische Feder oder Schneckenfeder ist, mit der der drehbar gelagerte Ventilkörper drehbar federnd gegen den Gaseinlass (8) gelagert ist.

7. Vakuummotor nach einem der vorangehenden Ansprüche, **dadurch gekennzeichnet, dass**
eine Stange (14) an einer Vorderseite des Arbeitskolbens (5) angeordnet ist, wobei die Vorderseite des Arbeitskolbens (5) der Vorderseite des Innenraums (4) zugewandt ist und wobei die Stange (14) durch die Vorderseite des Gehäuses (1, 2) hindurch aus dem Gehäuse (1, 2) herausragt und die Stange (14) in einer Führung in der Vorderseite des Gehäuses (1, 2) beweglich gelagert ist.

8. Vakuummotor nach Anspruch 7, **dadurch gekennzeichnet, dass** an der Vorderseite der Stange (14) ein Befestigungselement (26), insbesondere ein Gewinde (26), angeordnet ist, über das ein Werkzeug (36, 56, 58), wie eine Säge (36), eine Raspel (58) oder eine Bürste (56), mit einem zum Befestigungselement (26) passenden Gegenbefestigungselement, insbesondere einem zum Gewinde (26) passenden Gegengewinde, an der Stange (14) befestigbar ist.

9. Vakuummotor nach einem der vorangehenden Ansprüche, **dadurch gekennzeichnet, dass**
der wenigstens eine Stößel (12) an einer der Rückseite des Gehäuses (1, 2) zugewandten Rückseite des Arbeitskolbens (5) oder an einer dem Innenraum (4) zugewandten Vorderseite des Ventilkörpers (10) befestigt ist oder
der wenigstens eine Stößel (12) als separates Teil beweglich gegen das Gehäuse (1, 2) gelagert ist, vorzugsweise mit einem dritten Rückstellelement federnd gegen den Arbeitskolben (5) und den Ventilkörper (10) gelagert ist, oder
zumindest ein Stößel (12) an einer der Rückseite des Gehäuses (1, 2) zugewandten Rückseite des Arbeitskolbens (5) und zumindest ein weiterer Stößel an einer dem Innenraum (4) zugewandten Vorderseite des Ventilkörpers (10) befestigt ist,
wobei bevorzugt der wenigstens eine Stößel (12) oder die zumindest zwei Stößel (12) derart angeordnet ist oder sind, dass bei einer Bewegung des Arbeitskolbens (5) in Richtung der Rückseite des Gehäuses (1, 2) der Ventilkörper (10) über den wenigstens einen Stößel (12) oder über die zumindest zwei Stößel (12) in Richtung der Rückseite des Gehäuses (1, 2) gedrückt oder geschlagen wird.

10. Vakuummotor nach einem der vorangehenden Ansprüche, **dadurch gekennzeichnet, dass**
die Länge des wenigstens einen Stößels (12) oder die Summe der Längen zumindest eines Stößels (12) am Arbeitskolben (5) und zumindest eines Stößels (12) am Ventilkörper (10) kleiner ist als der Abstand zwischen dem Ventilkörper (10) in der geschlossenen Stellung und der Rückseite des Arbeitskolbens (5), wenn der Arbeitskolben (5) maximal in Richtung der Vorderseite des Innenraums (4) ausgelenkt ist.

11. Vakuummotor nach einem der vorangehenden Ansprüche, **dadurch gekennzeichnet, dass**
das erste Rückstellelement (6) eine Druckfeder (6) ist, die zwischen einer der Rückseite des Gehäuses (1, 2) zugewandte Rückseite des Arbeitskolbens (5) und der Rückseite des Innenraums (4) angeordnet ist, wobei die Druckfeder (6) den Arbeitskolben (5) in Richtung der Vorderseite des Innenraums (4) drückt.

12. Vakuummotor nach einem der vorangehenden Ansprüche, **dadurch gekennzeichnet, dass**
das Gehäuse (1, 2), der Arbeitskolben (5), der Ventilkörper (10) und die Stange (14) aus einem Kunststoff, insbesondere aus einem thermoplastischen Kunststoff gefertigt sind, vorzugsweise durch Spritzgießen geformt sind.

13. Vakuummotor nach einem der vorangehenden Ansprüche, **dadurch gekennzeichnet, dass**
der Ventilkörper (10) ein rotationssymmetrischer Körper ist, insbesondere ein Zylinder, ein Zylinder mit konischer Spitze oder ogivenförmiger Spitze, eine Kugel oder eine Ogive ist, wobei vorzugsweise die Dreh-Symmetrieachse des rotationssymmetrischen Körpers der Bewegungsrichtung des Ventilkörpers (10) entspricht.

14. Chirurgisches Antriebssystem aufweisend einen Vakuummotor nach einem der vorangehenden Ansprüche sowie ein manuell bedienbares Ventil (44), wobei das Ventil (44) in einer Leitung (42) angeordnet ist, die mit dem Gasauslass (7) und die mit der Unterdruckquelle verbindbar oder verbunden ist, so dass mit dem Ventil (44) die Verbindung des Gasauslasses (7) zur Unterdruckquelle unterbrechbar und/oder der Unterdruck am Gasauslass (7) einstellbar ist oder
das manuell bedienbare Ventil über eine Leitung mit dem Gaseinlass (8) verbunden ist, wobei die Leitung mit der Umgebung oder mit einer Druckgasquelle verbunden ist.

15. Chirurgisches Antriebssystem nach Anspruch 14, **dadurch gekennzeichnet, dass** das chirurgische Antriebssystem einen Griff (40) aufweist, mit dem es in einer Hand gehalten werden kann und das Ventil (44) mit einem Abzug (46) am Griff (40) bedienbar ist.

16. Chirurgisches Antriebssystem nach Anspruch 14 oder 15, **dadurch gekennzeichnet, dass**
das chirurgische Antriebssystem ein äußeres Gehäuse (38) aufweist, das das Gehäuse (1, 2) des Vakuummotors umschließt und das eine Führung aufweist, durch die die Stange (14) aus dem äußeren Gehäuse (1, 2) herausragt.

17. Medizinisches Lavage-System zum Debridement von Weichgewebe und/oder Knochengewebe aufweisend einen Vakuummotor nach einem der Ansprüche 1 bis 13 oder ein chirurgisches Antriebssystem nach einem der Ansprüche 14 bis 16 oder medizinische Vorrichtung zum Bürsten, Raspeln oder Sägen von Weichgewebe und/oder Knochengewebe aufweisend einen Vakuummotor nach einem der Ansprüche 1 bis 13 oder ein chirurgisches Antriebssystem nach einem der Ansprüche 14 bis 16.

18. Verfahren zum Betreiben eines Vakuummotors, bei dem ein Arbeitskolben (5) linear in einem zylindrischen Innenraum (4) oszilliert, der Innenraum (4) aufweisend eine Vorderseite und eine der Vorderseite gegenüberliegende Rückseite, wobei das Verfahren die folgenden Schritte umfasst:
A) Evakuieren von Gas aus dem Innenraum (4) zwischen dem Arbeitskolben (5) und der Rückseite des Innenraums (4) durch einen offenen Gasauslass (7),
B) Bewegen des Arbeitskolbens (5) in Richtung der Rückseite des Innenraums (4) aufgrund des Unterdrucks,
C) Spannen eines ersten Rückstellelements (6) durch die Bewegung des Arbeitskolbens (5),
D) Öffnen eines Gaseinlasses (8) im Bereich der Rückseite des Innenraums (4) durch die Bewegung des Arbeitskolbens (5), indem ein Impuls von dem Arbeitskolben (5) über wenigstens einen Stößel (12) auf einen beweglich gelagerten Ventilkörper (10) übertragen wird, wobei der Gaseinlass (8) durch die Bewegung des Ventilkörpers (10) geöffnet wird, wobei der freie Querschnitt des geöffneten Gaseinlasses (8) zumindest genauso groß ist wie der freie Querschnitt des Gasauslasses (7),
wobei zum Öffnen des Gaseinlasses (8) der Ventilkörper (10) gegen die Kraft eines zweiten Rückstellelements (11) bewegt wird und dabei das zweite Rückstellelement (11) gespannt wird, indem während der Bewegung des Arbeitskolbens (5) in Richtung der Rückseite des Innenraums (4) der Arbeitskolben (5) den Ventilkörper (10) mittels des wenigstens einen Stößels (12) am Ventilkörper (10) und/oder am Arbeitskolben (5) anstößt oder anschlägt und aus einem Ventilsitz stößt oder schlägt,
E) Einströmen von Umgebungsluft oder Druckgas in den Innenraum (4) zwischen dem Arbeitskolben (5) und der Rückseite des Innenraums (4) durch den Gaseinlass (8),
F) Bewegen des Arbeitskolbens (5) in Richtung der Vorderseite des Innenraums (4) angetrieben von dem gespannten ersten Rückstellelement (6), wobei dabei eine Stange (14), die an dem Arbeitskolben (5) befestigt ist und durch eine Führung aus der Vorderseite des Innenraums (4) herausgeführt ist, aus dem Innenraum (4) herausgedrückt wird,
G) Verschließen des Gaseinlasses (8) durch eine umgekehrte Bewegung des Ventilkörpers (10) angetrieben durch das zweite Rückstellelement (11).

## Claims

1. A vacuum motor having
a housing (1, 2) comprising a cylindrical internal space (4), wherein the housing (1, 2) and the internal space (4) each have a front side and a rear side located opposite the front side, wherein the front side of the internal space (4) faces the front side of the housing (1, 2),
a working piston (5), which is movably arranged so as to oscillate linearly between the front side and the rear side of the internal space (4) in the axial direction of the cylinder axis of the cylindrical inner space (4),
a first restoring element (6), which is arranged in the internal space (4) and which at least temporarily exerts a force on the working piston (5) that acts in the direction of the front side of the internal space (4),
a gas outlet (7) in the housing (1, 2) for discharging the gas from the internal space (4), wherein the gas outlet (7) can be connected or is connected to a negative pressure source,
a gas inlet (8) in the housing (1, 2) for feeding ambient air or a pressurized gas into the internal space (4), wherein the gas outlet (7) and the gas inlet (8) end in the internal space (4) between the working piston (5) and the rear side of the housing (1, 2), and wherein the working piston (5) does not completely cover or even only partially covers the gas outlet (7) and the gas inlet (8) in any position,
a valve body (10), which can be moved against the gas inlet (8), for closing the gas inlet (8),
at least one plunger (12), which is movably arranged in the axial direction within the housing (1, 2) between the working piston (5) and the valve body (10),
**characterised by**
a second restoring element (11), which transfers the valve body (10) into the closed position, so that the valve body (10) closes the gas inlet (8), wherein an impulse is transferred from the working piston (5) via the at least one plunger (12) to the valve body (10) in response to a movement of the working piston (5) in the direction of the rear side of the internal space (4),
so that the valve body (10) is moved against the force of the second restoring element (11), and the gas inlet (8) is thus opened, and wherein the free cross section of the opened gas inlet (8) is at least just as large as the free cross section of the gas outlet (7).

2. The vacuum motor according to claim 1, **characterised in that** the valve body (10) cannot close the gas outlet (7) in any position.

3. The vacuum motor according to claim 1 or 2, **characterised in that** a front part of the internal space (4) is connected via at least one ventilation opening (32) in the housing (1, 2) to the surrounding area of the housing (1, 2), wherein the front part of the internal space (4) is separated from the gas outlet (7) by the working piston (5) in every position of the working piston (5).

4. The vacuum motor according to any one of claims 1 to 3, **characterised in that** the second restoring element (11) is an elastic spring (11), by means of which the linearly movable valve body (10) is supported against the gas inlet (8) in a linearly spring-loaded manner.

5. The vacuum motor according to any one of the preceding claims, **characterised in that**
the gas inlet (8) is arranged on the rear side of the internal space (4) and that the gas inlet (8) has a valve element comprising a cylindrical valve space (20) and comprising a feedthrough (22), which can be closed by the valve body (10) and which, in the open state, connects the internal space (4) and the cylindrical valve space (20),
wherein the valve body (10) is guided and movably supported in the cylindrical valve space (20) in the axial direction of the cylindrical valve space (20), and the cylindrical valve space (20) has a gas inlet opening (9) for feeding ambient air or a pressurized gas, wherein the second restoring element (11) is preferably an elastic spring (11), which is arranged between the valve body (10) and a rear side of the valve space (20) facing away from the internal space (4).

6. A vacuum motor according to any one of claims 1 to 3, **characterised in that** the valve body is rotatably supported against the gas inlet (8), and the second restoring element is an elastic spring or coil spring, by means of which the rotatably supported valve body is rotatably supported against the gas inlet (8) in a spring-loaded manner.

7. The vacuum motor according to any one of the preceding claims, **characterised in that**
a rod (14) is arranged on a front side of the working piston (5), wherein the front side of the working piston (5) faces the front side of the internal space (4), and wherein the rod (14) protrudes from the housing (1, 2) through the front side of the housing (1, 2), and the rod (14) is movably supported in a guide in the front side of the housing (1, 2).

8. The vacuum motor according to claim 7, **characterised in that**
a fastening element (26), in particular a thread (26), is arranged on the front side of the rod (14), via which a tool (36, 56, 58), such as a saw (36), a rasp (58), or a brush (56), comprising a counter fastening element compatible with the fastening element (26), in particular a counter thread compatible with the thread (26), can be fastened to the rod (14).

9. The vacuum motor according to any one of the preceding claims, **characterised in that**
the at least one plunger (12) is fastened to a rear side of the working piston (5) facing the rear side of the housing (1, 2) or to a front side of the valve body (10) facing the internal space (4), or
the at least one plunger (12) is movably supported against the housing (1, 2) as separate part, is preferably supported against the working piston (5) and the valve body (10) by means of a third restoring element in a spring-loaded manner, or
at least one plunger (12) is fastened to a rear side of the working piston (5) facing the rear side of the housing (1, 2), and at least a further plunger is fastened to a front side of the valve body (10) facing the internal space (4), wherein the at least one plunger (12) or the at least two plungers (12) is or are preferably arranged in such a way that the valve body (10) is pushed or hit in the direction of the rear side of the housing (1, 2) via the at least one plunger (12) or via the at least two plungers (12) in response to a movement of the working piston (5) in the direction of the rear side of the housing (1, 2).

10. The vacuum motor according to any one of the preceding claims, **characterised in that**
the length of the at least one plunger (12) or the sum of the lengths of at least one plunger (12) at the working piston (5) and of at least one plunger (12) at the valve body (10) is smaller than the distance between the valve body (10) in the closed position and the rear side of the working piston (5), when the working piston (5) is maximally deflected in the direction of the front side of the internal space (4).

11. The vacuum motor according to any one of the preceding claims, **characterised in that**
the first restoring element (6) is a pressure spring (6), which is arranged between a rear side of the working piston (5) facing the rear side of the housing (1, 2), and the rear side of the internal space (4), wherein the pressure spring (6) pushes the working piston (5) in the direction of the front side of the internal space (4).

12. The vacuum motor according to any one of the preceding claims, **characterised in that**
the housing (1, 2), the working piston (5), the valve body (10), and the rod (14) are made of a plastic, in particular of a thermoplastic plastic, preferably formed by injection moulding.

13. The vacuum motor according to any one of the preceding claims, **characterised in that**
the valve body (10) is a rotationally symmetrical body, in particular a cylinder, a cylinder comprising conical tip or ogive-shaped tip, a sphere or an ogive, wherein the axis of rotational symmetry of the rotationally symmetrical body preferably corresponds to the direction of movement of the valve body (10).

14. A surgical drive system having a vacuum motor according to any one of the preceding claims as well as a manually operable valve (44), wherein the valve (44) is arranged in a line (42), which can be connected or is connected to the gas outlet (7) and to the negative pressure source, so that the connection of the gas outlet (7) to the negative pressure source can be interrupted and/or the negative pressure at the gas outlet (7) can be set by means of the valve (44), or
the manually operable valve is connected to the gas inlet (8) via a line, wherein the line is connected to the surrounding area or to a compressed gas source.

15. The surgical drive system according to claim 14, **characterised in that** the surgical drive system has a handle (40), by means of which it can be held in one hand, and the valve (44) can be operated by means of a trigger (46) on the handle (40).

16. The surgical drive system according to claim 14 or 15, **characterised in that** the surgical drive system has an outer housing (38), which surrounds the housing (1, 2) of the vacuum motor and which has a guide, through which the rod (14) protrudes from the outer housing (1, 2).

17. A medical lavage system for debridement of soft tissue and/or bone tissue having a vacuum motor according to any one of claims 1 to 13, or a surgical drive system according to any one of claims 14 to 16, or medial device for brushing, rasping, or sawing soft tissue and/or bone tissue, having a vacuum motor according to any one of claims 1 to 13, or a surgical drive system according to any one of claims 14 to 16.

18. A method for operating a vacuum motor, in the case of which a working piston (5) oscillates linearly in a cylindrical internal space (4), the internal space (4) having a front side and a rear side located opposite the front side, wherein the method comprises the following steps:
A) evacuating gas from the internal space (4) between the working piston (5) and the rear side of the internal space (4) through an open gas outlet (7),
B) moving the working piston (5) in the direction of the rear side of the internal space (4) due to the negative pressure,
C) tensioning a first restoring element (6) by the movement of the working piston (5),
D) opening a gas inlet (8) in the area of the rear side of the internal space (4) by the movement of the working piston (5), in that an impulse is transferred from the working piston (5) via at least one plunger (12) to a movably supported valve body (10), wherein the gas inlet (8) is opened by the movement of the valve body (10), wherein the free cross section of the opened gas inlet (8) is at least just as large as the free cross section of the gas outlet (7),
wherein, to open the gas inlet (8), the valve body (10) is moved against the force of a second restoring element (11), and the second restoring element (11) is tensioned thereby, in that the working piston (5) pushes or hits against the valve body (10) and/or against the working piston (5) by means of at least one plunger (12) and pushes or hits the valve body out of a valve seat during the movement of the working piston (5) in the direction of the rear side of the internal space (4),
E) inflow of ambient air or compressed gas into the internal space (4) between the working piston (5) and the rear side of the internal space (4) through the gas inlet (8),
F) moving the working piston (5) in the direction of the front side of the internal space (4) driven by the tensioned first restoring element (6), wherein a rod (14), which is fastened to the working piston (5) and which is guided out of the front side of the internal space (4) through a guide, is thereby pushed out of the internal space (4),
G) closing the gas inlet (8) by a reverse movement of the valve body (10) driven by the second restoring element (11).

## Revendications

1. Moteur à vide présentant
un carter (1, 2) avec un espace intérieur (4) cylindrique, le carter (1, 2) et l'espace intérieur (4) cylindrique ayant respectivement une face avant et une face arrière opposée à la face avant, la face avant de l'espace intérieur (4) étant tournée vers la face avant du carter (1, 2),
un piston de travail (5) qui est disposé linéairement en mouvement oscillant entre la face avant et la face arrière de l'espace intérieur (4) dans le sens axial de l'axe du cylindre de l'espace intérieur (4) cylindrique,
un premier élément de rappel (6) qui est disposé dans l'espace intérieur (4) et qui exerce, au moins temporairement, une force agissant en direction de la face avant de l'espace intérieur (4) sur le piston de travail (5),
une sortie de gaz (7) dans le carter (1, 2) pour évacuer le gaz de l'espace intérieur (4), la sortie de gaz (7) étant reliable ou relié à une source de dépression,
une entrée de gaz (8) dans le carter (1, 2) pour injecter de l'air ambiant ou un gaz sous pression dans l'espace intérieur (4), la sortie de gaz (7) et l'entrée de gaz (8) conduisant dans l'espace intérieur (4) entre le piston de travail (5) et la face arrière du carter (1, 2), et le piston de travail (5) ne couvrant complètement la sortie de gaz (7) en aucune position ou ne la couvrant que partiellement en aucune position,
un corps de vanne (10) déplaçable contre l'entrée de gaz (8) pour obturer l'entrée de gaz (8),
au moins un poussoir (12) qui est mobile dans le sens axial à l'intérieur du carter (1, 2) entre le piston de travail (5) et le corps de vanne (10), **caractérisé en ce que**
un deuxième élément de rappel (11) qui transfère le corps de vanne (10) en position fermée de façon à ce que le corps de vanne (10) obture l'entrée de gaz (8), une impulsion étant transmise sur le corps de vanne (10) par le piston de travail (5) lors d'un déplacement du piston de travail (5) en direction de la face arrière de l'espace intérieur (4) par au moins un poussoir (12),
de façon à ce que le corps de vanne (10) soit déplacé contre la force du deuxième élément de rappel (11),
et que l'entrée de gaz (8) s'ouvre ainsi,
et la section libre de l'entrée de gaz (8) ouverte étant au moins aussi grande que la section libre de la sortie de gaz (7).

2. Moteur à vide conformément à la revendication n°1, **caractérisé en ce que** le corps de vanne (10) ne peut obturer la sortie de gaz (7) en aucune position.

3. Moteur à vide conformément à la revendication n°1 ou n°2, **caractérisé en ce que**
une partie frontale de l'espace intérieur (4) est reliée à l'environnement du carter (1, 2) par au moins un orifice de ventilation (32) dans le carter (1, 2), la partie frontale de l'espace intérieur (4) étant séparée de la sortie de gaz (7) par le piston de travail (5) dans chaque position du piston de travail (5).

4. Moteur à vide conformément à l'une des revendications n°1 à n°3, **caractérisé en ce que**
le deuxième élément de rappel (11) est un ressort élastique (11) avec lequel le corps de vanne (10) déplaçable linéairement est monté linéairement et élastiquement contre l'entrée de gaz (8).

5. Moteur à vide conformément à l'une des revendications précédentes, **caractérisé en ce que**
l'entrée de gaz (8) est disposée sur la face arrière de l'espace intérieur (4) et que l'entrée de gaz (8) présente un élément de vanne avec un espace de vanne (20) cylindrique et un passage (22) reliant l'espace intérieur (4) et l'espace de vanne (20) cylindrique à l'état ouvert et obturable par le corps de vanne (10), le corps de vanne (10) dans l'espace de vanne (20) cylindrique étant guidé dans le sens axial de l'espace de vanne (20) cylindrique et pouvant être déplacé et l'espace de vanne (20) cylindrique présentant un orifice d'admission de gaz (9) pour injecter de l'air ambiant ou un gaz sous pression, le deuxième élément de rappel (11) étant préférablement un ressort élastique (11) qui est disposé entre le corps de vanne (10) et une face arrière de l'espace de vanne (20), détournée de l'espace intérieur (4).

6. Moteur à vide conformément à l'une des revendications n°1 à n°3, **caractérisé en ce que**
le corps de vanne est pivotant contre l'entrée de gaz (8) et que le deuxième élément de rappel est un ressort élastique ou un ressort conique avec lequel le corps de vanne pivotant est pivotant élastiquement contre l'entrée de gaz (8).

7. Moteur à vide conformément à l'une des revendications précédentes, **caractérisé en ce que**
une tige (14) est disposée sur une face avant du piston de travail (5), la face avant du piston de travail (5) étant tournée vers la face avant de l'espace intérieur (4), la tige (14) dépassant du carter (1, 2) en passant par la face avant du carter (1, 2) et la tige (14) étant déplaçable dans un guidage dans la face avant du carter (1, 2).

8. Moteur à vide conformément à la revendication n°7, **caractérisé en ce que** un élément de fixation (26), en particulier un filetage (26), par lequel un outil (36, 56, 58), comme une scie (36), une râpe (58) ou une brosse (56), peut être fixé sur la tige (14) avec un élément de contre-fixation adapté à l'élément de fixation (26), en particulier un contre-filetage adapté au filetage (26), est disposé sur la face avant de la tige (14).

9. Moteur à vide conformément à l'une des revendications précédentes, **caractérisé en ce que**
au moins un poussoir (12) est fixé sur une face arrière du piston de travail (5), tournée vers la face arrière du carter (1, 2), ou sur une face avant du corps de vanne (10), tournée vers l'espace intérieur (4), ou
au moins un poussoir (12) est déplaçable contre le carter (1, 2) en tant que pièce séparée, est préférablement monté sur ressort contre le piston de travail (5) et le corps de vanne (10) avec un troisième élément de rappel ou
au moins un poussoir (12) est fixé sur une face arrière du piston de travail (5), tournée vers la face arrière du carter (1, 2), et au moins un autre poussoir est fixé sur une face avant du corps de vanne (10), tournée vers l'espace intérieur (4),
préférablement au moins un poussoir (12) est disposé ou au moins deux poussoirs (12) sont disposés de telle manière à ce que, lors d'un déplacement du piston de travail (5) en direction de la face arrière du carter (1, 2), le corps de vanne (10) soit poussé ou frappé en direction de la face arrière du carter (1, 2) par au moins un poussoir (12) ou au moins deux poussoirs (12).

10. Moteur à vide conformément à l'une des revendications précédentes, **caractérisé en ce que**
la longueur au moins d'un poussoir (12) ou la somme des longueurs au moins d'un poussoir (12) sur le piston de travail (5) et au moins d'un poussoir (12) sur le corps de vanne (10) est inférieure à la distance entre le corps de vanne (10) en position fermée et la face arrière du piston de travail (5) lorsque le piston de travail (5) est dévié au maximum en direction de la face avant de l'espace intérieur (4).

11. Moteur à vide conformément à l'une des revendications précédentes, **caractérisé en ce que**
le premier élément de rappel (6) est un ressort de compression (6) qui est disposé entre une face arrière du piston de travail (5), tournée vers la face arrière du carter (1, 2), et la face arrière de l'espace intérieur (4), le ressort de compression (6) poussant le piston de travail (5) en direction de la face avant de l'espace intérieur (4).

12. Moteur à vide conformément à l'une des revendications précédentes, **caractérisé en ce que**
le carter (1, 2), le piston de travail (5), le corps de vanne (10) et la tige (14) sont réalisés dans une matière plastique, en particulier dans une matière thermoplastique, et sont préférablement formés par moulage par injection.

13. Moteur à vide conformément à l'une des revendications précédentes, **caractérisé en ce que**
le corps de vanne (10) est un corps symétrique en rotation, en particulier un cylindre, un cylindre à pointe conique ou à pointe en forme d'ogive, une boule ou une ogive, l'axe de symétrie rotationnelle du corps symétrique en rotation correspondant préférablement au sens de déplacement du corps de vanne (10).

14. Système de commande chirurgical présentant un moteur à vide conformément à l'une des revendications précédentes ainsi qu'une vanne à commande manuelle (44), la vanne (44) étant disposée dans une conduite (42) qui est reliable ou reliée à la sortie de gaz (7) et à la source de dépression de façon à ce que la liaison de la sortie de gaz (7) vers la source de dépression puisse être interrompue et/ou que la dépression soit réglable à la sortie de gaz (7) avec la vanne (44) ou que la vanne à commande manuelle soit reliée à l'entrée de gaz (8) par une conduite, la conduite étant reliée à l'environnement ou à une source de gaz sous pression.

15. Système de commande chirurgical conformément à la revendication n°14, **caractérisé en ce que**
le système de commande chirurgical présente une poignée (40) permettant de le tenir dans une main et que la vanne (44) peut être actionnée au moyen d'un système de détente (46) sur la poignée (40).

16. Système de commande chirurgical conformément à la revendication n°14 ou n°15, **caractérisé en ce que**
le système de commande chirurgical présente un carter extérieur (38) qui renferme le carter (1, 2) du moteur à vide et qui présente un guidage par lequel la tige (14) dépasse du carter (1, 2) extérieur.

17. Système de lavage médical pour le débridement de tissus mous et/ou de tissus osseux, présentant un moteur à vide conformément à l'une des revendications n°1 à n°13 ou un système de commande chirurgical conformément à l'une des revendications n°14 à n°16 ou un dispositif médical pour le brossage, le râpage ou le sciage de tissus mous et/ou de tissus osseux, présentant un moteur à vide conformément à l'une des revendications n°1 à n°13 ou un système de commande chirurgical conformément à l'une des revendications n°14 à n°16.

18. Procédé pour l'exploitation d'un moteur à vide, sur lequel un piston de travail (5) oscille linéairement dans un espace intérieur (4) cylindrique, l'espace intérieur (4) présentant une face avant et une face arrière opposée à la face avant, le procédé comprenant les étapes suivantes :
A) Évacuation du gaz de l'espace intérieur (4) entre le piston de travail (5) et la face arrière de l'espace intérieur (4) par une sortie de gaz (7) ouverte
B) Déplacement du piston de travail (5) en direction de la face arrière de l'espace intérieur (4) en raison de la dépression
C) Tension d'un premier élément de rappel (6) par le déplacement du piston de travail (5)
D) Ouverture d'une entrée de gaz (8) dans la zone de la face arrière de l'espace intérieur (4) par le déplacement du piston de travail (5) par la transmission d'une impulsion du piston de travail (5) sur un corps de vanne (10) déplaçable par au moins un poussoir (12), l'entrée de gaz (8) étant ouverte par le déplacement du corps de vanne (10), la section libre de l'entrée de gaz (8) ouverte étant au moins aussi grande que la section libre de la sortie de gaz (7),
le corps de vanne (10) étant déplacé contre la force d'un deuxième élément de rappel (11) pour l'ouverture de l'entrée de gaz (8) et le deuxième élément de rappel (11) étant tendu, par le fait que, pendant le déplacement du piston de travail (5) en direction de la face arrière de l'espace intérieur (4), le piston de travail (5) pousse ou frappe le corps de vanne (10) au moyen au moins d'un poussoir (12) sur le corps de vanne (10) et/ou sur le piston de travail (5) et le pousse ou le frappe hors d'un siège de vanne,
E) Afflux d'air ambiant ou de gaz sous pression dans l'espace intérieur (4) entre le piston de travail (5) et la face arrière de l'espace intérieur (4) par l'entrée de gaz (8),
F) Déplacement du piston de travail (5) en direction de la face avant de l'espace intérieur (4), actionné par le premier élément de rappel (6) tendu, une tige (14) qui est fixée sur le piston de travail (5) et qui est sortie de la face avant de l'espace intérieur (4) par un guidage, étant extraite de l'espace intérieur (4),
G) Obturation de l'entrée de gaz (8) par un déplacement inversé du corps de vanne (10), actionné par le deuxième élément de rappel (11).
